# EUROPEAN PATENT SPECIFICATION

(11) **EP 2 363 461 B1**
(45) Date of publication and mention of the grant of the patent: **24.12.2014**
(21) Application number: 09827488.9
(22) Date of filing: 06.11.2009
(51) Int. Cl.: C12N 15/00, C07K 19/00, C12N 1/21, C12N 5/071, C12N 9/52, C12N 15/09, C07K 14/78

(54) **FUSION COLLAGENASE TO WHICH AFFINITY TAG IS ATTACHED, AND METHOD FOR PRODUCING SAME**
FUSIONS-COLLAGENASE MIT DARAN GEBUNDENEM AFFINITÄTS-TAG UND VERFAHREN ZUR HERSTELLUNG DAVON
COLLAGÉNASE DE FUSION À LAQUELLE UN MARQUEUR D'AFFINITÉ EST FIXÉ ET SON PROCÉDÉ DE FABRICATION

(30) Priority: 19.11.2008 JP 2008295922
(43) Date of publication of application: 07.09.2011
(73) Proprietor: Meiji Seika Pharma Co., Ltd., Tokyo 104-8002 (JP); Tohoku University, Aoba-ku Sendai-shi Miyagi 980-8577 (JP)
(72) Inventor: FUKUSHIMA Takayoshi, Odawara-shi Kanagawa 250-0852 (JP); YOKOYAMA Kengo, Odawara-shi Kanagawa 250-0852 (JP); MURASHIMA Koichiro, Odawara-shi Kanagawa 250-0852 (JP); GOTO Masafumi, Sendai-shi Miyagi 980-8577 (JP); YAMAGATA Youhei, Sendai-shi Miyagi 980-8577 (JP)
(74) Representative: Cohausz & Florack
(86) International application number: PCT/JP2009/068968
(87) International publication number: WO 2010/058707

(56) References cited:
- WO-A2-2008/124166
- JP-A- 2003 284 553
- SAGAYA THERESA LEENA PHILOMINATHAN ET AL: "1H, 13C and 15N resonance assignments of Ca2+ bound collagen-binding domain derived from a clostridial collagenase", BIOMOLECULAR NMR ASSIGNMENTS, vol. 2, no. 2, 22 July 2008 (2008-07-22), pages 127-129, XP55030622, ISSN: 1874-2718, DOI: 10.1007/s12104-008-9102-z
- TAMAI E ET AL.: 'High-level expression of his- tagged clostridial collagenase in Clostridium perfringens.' APPL MICROBIOL BIOTECHNOL. vol. 80, no. 4, September 2008, pages 627 - 35, XP019623735
- MATSUSHITA 0 ET AL.: 'Gene duplication and multiplicity of collagenases in Clostridium histolyticum.' J BACTERIOL. vol. 181, no. 3, 1999, pages 923 - 33, XP001147203
- YOSHIHARA K ET AL.: 'Cloning and nucleotide sequence analysis of the colH gene from Clostridium histolyticum encoding a collagenase and a gelatinase.' J BACTERIOL. vol. 176, no. 21, 1994, pages 6489 - 96, XP008150460
- YOSHITOMO OKA: 'Tokushu/Saibo Baiyo Saibo Baiyo wa dokomade Kano ka, 2) Suito Saibo' BYOTAI SEIRI vol. 6, no. 11, 1987, pages 852 - 856, XP008150462

SEQUENCE LISTING
<110> MEIJI SEIKA KAISHA, LTD.
   TOHOKU UNIVERSITY
<120> A Collagenase with an affinity tag, and its preparation method
<130> M0833
<150> JP 2008-295922
   <151> 2008-11-19
<160> 16
<170> PatentIn version 3.1
<210> 1
   <211> 1118
   <212> PRT
   <213> Clostridium histolyticum
<220>
   <221> SIGNAL
   <222> (-110)..(-1)
   <223>
<220>
   <221> mat_peptide
   <222> (1)..(1008)
   <223>
<400> 1
   Met Lys Lys Asn Ile Leu Lys Ile Leu Met Asp Ser Tyr Ser Lys
   -110 -105 -100
   Glu Ser Lys Ile Gln Thr Val Arg Arg Val Thr Ser Val Ser Leu Leu
   -95 -90 -85 -80
   Ala Val Tyr Leu Thr Met Asn Thr Ser Ser Leu Val Leu Ala Lys Pro
   -75 -70 -65
   Ile Glu Asn Thr Asn Asp Thr Ser Ile Lys Asn Val Glu Lys Leu Arg
   -60 -55 -50
   Asn Ala Pro Asn Glu Glu Asn Ser Lys Lys Val Glu Asp Ser Lys Asn
   -45 -40 -35
   Asp Lys Val Glu His Val Lys Asn Ile Glu Glu Ala Lys Val Glu Gln
   -30 -25 -20
   Val Ala Pro Glu Val Lys Ser Lys Ser Thr Leu Arg Ser Ala Ser Ile
   -15 -10 -5 -1 1
   Ala Asn Thr Asn Ser Glu Lys Tyr Asp Phe Glu Tyr Leu Asn Gly Leu
   5 10 15
   Ser Tyr Thr Glu Leu Thr Asn Leu Ile Lys Asn Ile Lys Trp Asn Gln
   20 25 30
   Ile Asn Gly Leu Phe Asn Tyr Ser Thr Gly Ser Gln Lys Phe Phe Gly
   35 40 45
   Asp Lys Asn Arg Val Gln Ala Ile Ile Asn Ala Leu Gln Glu Ser Gly
   50 55 60 65
   Arg Thr Tyr Thr Ala Asn Asp Met Lys Gly Ile Glu Thr Phe Thr Glu
   70 75 80
   Val Leu Arg Ala Gly Phe Tyr Leu Gly Tyr Tyr Asn Asp Gly Leu Ser
   85 90 95
   Tyr Leu Asn Asp Arg Asn Phe Gln Asp Lys Cys Ile Pro Ala Met Ile
   100 105 110
   Ala Ile Gln Lys Asn Pro Asn Phe Lys Leu Gly Thr Ala Val Gln Asp
   115 120 125
   Glu Val Ile Thr Ser Leu Gly Lys Leu Ile Gly Asn Ala Ser Ala Asn
   130 135 140 145
   Ala Glu Val Val Asn Asn Cys Val Pro Val Leu Lys Gln Phe Arg Glu
   150 155 160
   Asn Leu Asn Gln Tyr Ala Pro Asp Tyr Val Lys Gly Thr Ala Val Asn
   165 170 175
   Glu Leu Ile Lys Gly Ile Glu Phe Asp Phe Ser Gly Ala Ala Tyr Glu
   180 185 190
   Lys Asp Val Lys Thr Met Pro Trp Tyr Gly Lys Ile Asp Pro Phe Ile
   195 200 205
   Asn Glu Leu Lys Ala Leu Gly Leu Tyr Gly Asn Ile Thr Ser Ala Thr
   210 215 220 225
   Glu Trp Ala Ser Asp Val Gly Ile Tyr Tyr Leu Ser Lys Phe Gly Leu
   230 235 240
   Tyr Ser Thr Asn Arg Asn Asp Ile Val Gln Ser Leu Glu Lys Ala Val
   245 250 255
   Asp Met Tyr Lys Tyr Gly Lys Ile Ala Phe Val Ala Met Glu Arg Ile
   260 265 270
   Thr Trp Asp Tyr Asp Gly Ile Gly Ser Asn Gly Lys Lys Val Asp His
   275 280 285
   Asp Lys Phe Leu Asp Asp Ala Glu Lys His Tyr Leu Pro Lys Thr Tyr
   290 295 300 305
   Thr Phe Asp Asn Gly Thr Phe Ile Ile Arg Ala Gly Asp Lys Val Ser
   310 315 320
   Glu Glu Lys Ile Lys Arg Leu Tyr Trp Ala Ser Arg Glu Val Lys Ser
   325 330 335
   Gln Phe His Arg Val Val Gly Asn Asp Lys Ala Leu Glu Val Gly Asn
   340 345 350
   Ala Asp Asp Val Leu Thr Met Lys Ile Phe Asn Ser Pro Glu Glu Tyr
   355 360 365
   Lys Phe Asn Thr Asn Ile Asn Gly Val Ser Thr Asp Asn Gly Gly Leu
   370 375 380 385
   Tyr Ile Glu Pro Arg Gly Thr Phe Tyr Thr Tyr Glu Arg Thr Pro Gln
   390 395 400
   Gln Ser Ile Phe Ser Leu Glu Glu Leu Phe Arg His Glu Tyr Thr His
   405 410 415
   Tyr Leu Gln Ala Arg Tyr Leu Val Asp Gly Leu Trp Gly Gln Gly Pro
   420 425 430
   Phe Tyr Glu Lys Asn Arg Leu Thr Trp Phe Asp Glu Gly Thr Ala Glu
   435 440 445
   Phe Phe Ala Gly Ser Thr Arg Thr Ser Gly Val Leu Pro Arg Lys Ser
   450 455 460 465
   Ile Leu Gly Tyr Leu Ala Lys Asp Lys Val Asp His Arg Tyr Ser Leu
   470 475 480
   Lys Lys Thr Leu Asn Ser Gly Tyr Asp Asp Ser Asp Trp Met Phe Tyr
   485 490 495
   Asn Tyr Gly Phe Ala Val Ala His Tyr Leu Tyr Glu Lys Asp Met Pro
   500 505 510
   Thr Phe Ile Lys Met Asn Lys Ala Ile Leu Asn Thr Asp Val Lys Ser
   515 520 525
   Tyr Asp Glu Ile Ile Lys Lys Leu Ser Asp Asp Ala Asn Lys Asn Thr
   530 535 540 545
   Glu Tyr Gln Asn His Ile Gln Glu Leu Ala Asp Lys Tyr Gln Gly Ala
   550 555 560
   Gly Ile Pro Leu Val Ser Asp Asp Tyr Leu Lys Asp His Gly Tyr Lys
   565 570 575
   Lys Ala Ser Glu Val Tyr Ser Glu Ile Ser Lys Ala Ala Ser Leu Thr
   580 585 590
   Asn Thr Ser Val Thr Ala Glu Lys Ser Gln Tyr Phe Asn Thr Phe Thr
   595 600 605
   Leu Arg Gly Thr Tyr Thr Gly Glu Thr Ser Lys Gly Glu Phe Lys Asp
   610 615 620 625
   Trp Asp Glu Met Ser Lys Lys Leu Asp Gly Thr Leu Glu Ser Leu Ala
   630 635 640
   Lys Asn Ser Trp Ser Gly Tyr Lys Thr Leu Thr Ala Tyr Phe Thr Asn
   645 650 655
   Tyr Arg Val Thr Ser Asp Asn Lys Val Gln Tyr Asp Val Val Phe His
   660 665 670
   Gly Val Leu Thr Asp Asn Ala Asp Ile Ser Asn Asn Lys Ala Pro Ile
   675 680 685
   Ala Lys Val Thr Gly Pro Ser Thr Gly Ala Val Gly Arg Asn Ile Glu
   690 695 700 705
   Phe Ser Gly Lys Asp Ser Lys Asp Glu Asp Gly Lys Ile Val Ser Tyr
   710 715 720
   Asp Trp Asp Phe Gly Asp Gly Ala Thr Ser Arg Gly Lys Asn Ser Val
   725 730 735
   His Ala Tyr Lys Lys Ala Gly Thr Tyr Asn Val Thr Leu Lys Val Thr
   740 745 750
   Asp Asp Lys Gly Ala Thr Ala Thr Glu Ser Phe Thr Ile Glu Ile Lys
   755 760 765
   Asn Glu Asp Thr Thr Thr Pro Ile Thr Lys Glu Met Glu Pro Asn Asp
   770 775 780 785
   Asp Ile Lys Glu Ala Asn Gly Pro Ile Val Glu Gly Val Thr Val Lys
   790 795 800
   Gly Asp Leu Asn Gly Ser Asp Asp Ala Asp Thr Phe Tyr Phe Asp Val
   805 810 815
   Lys Glu Asp Gly Asp Val Thr Ile Glu Leu Pro Tyr Ser Gly Ser Ser
   820 825 830
   Asn Phe Thr Trp Leu Val Tyr Lys Glu Gly Asp Asp Gln Asn His Ile
   835 840 845
   Ala Ser Gly Ile Asp Lys Asn Asn Ser Lys Val Gly Thr Phe Lys Ser
   850 855 860 865
   Thr Lys Gly Arg His Tyr Val Phe Ile Tyr Lys His Asp Ser Ala Ser
   870 875 880
   Asn Ile Ser Tyr Ser Leu Asn Ile Lys Gly Leu Gly Asn Glu Lys Leu
   885 890 895
   Lys Glu Lys Glu Asn Asn Asp Ser Ser Asp Lys Ala Thr Val Ile Pro
   900 905 910
   Asn Phe Asn Thr Thr Met Gln Gly Ser Leu Leu Gly Asp Asp Ser Arg
   915 920 925
   Asp Tyr Tyr Ser Phe Glu Val Lys Glu Glu Gly Glu Val Asn Ile Glu
   930 935 940 945
   Leu Asp Lys Lys Asp Glu Phe Gly Val Thr Trp Thr Leu His Pro Glu
   950 955 960
   Ser Asn Ile Asn Asp Arg Ile Thr Tyr Gly Gln Val Asp Gly Asn Lys
   965 970 975
   Val Ser Asn Lys Val Lys Leu Arg Pro Gly Lys Tyr Tyr Leu Leu Val
   980 985 990
   Tyr Lys Tyr Ser Gly Ser Gly Asn Tyr Glu Leu Arg Val Asn Lys
   995 1000 1005
<210> 2
   <211> 1118
   <212> PRT
   <213> Clostridium histolyticum
<220>
   <221> SIGNAL
   <222> (-110)..(-1)
<220>
   <221> mat_peptide
   <222> (1)..(1008)
<400> 2
   Met Lys Lys Asn Ile Leu Lys Ile Leu Met Asp Ser Tyr Ser Lys
   -110 -105 -100
   Glu Ser Lys Ile Gln Thr Val Arg Arg Val Thr Ser Val Ser Leu Leu
   -95 -90 -85 -80
   Ala Val Tyr Leu Thr Met Asn Thr Ser Ser Leu Val Leu Ala Lys Pro
   -75 -70 -65
   Ile Glu Asn Thr Asn Asp Thr Ser Ile Lys Asn Val Glu Lys Leu Arg
   -60 -55 -50
   Asn Ala Pro Asn Glu Glu Asn Ser Lys Lys Val Glu Asp Ser Lys Asn
   -45 -40 -35
   Asp Lys Val Glu His Val Lys Asn Ile Glu Glu Ala Lys Val Glu Gln
   -30 -25 -20
   Val Ala Pro Glu Val Lys Ser Lys Ser Thr Leu Arg Ser Ala Ser Ile
   -15 -10 -5 -1 1
   Ala Asn Thr Asn Ser Glu Lys Tyr Asp Phe Glu Tyr Leu Asn Gly Leu
   5 10 15
   Ser Tyr Thr Glu Leu Thr Asn Leu Ile Lys Asn Ile Lys Trp Asn Gln
   20 25 30
   Ile Asn Gly Leu Phe Asn Tyr Ser Thr Gly Ser Gln Lys Phe Phe Gly
   35 40 45
   Asp Lys Asn Arg Val Gln Ala Ile Ile Asn Ala Leu Gln Glu Ser Gly
   50 55 60 65
   Arg Thr Tyr Thr Ala Asn Asp Met Lys Gly Ile Glu Thr Phe Thr Glu
   70 75 80
   Val Leu Arg Ala Gly Phe Tyr Leu Gly Tyr Tyr Asn Asp Gly Leu Ser
   85 90 95
   Tyr Leu Asn Asp Arg Asn Phe Gln Asp Lys Cys Ile Pro Ala Met Ile
   100 105 110
   Ala Ile Gln Lys Asn Pro Asn Phe Lys Leu Gly Thr Ala Val Gln Asp
   115 120 125
   Glu Val Ile Thr Ser Leu Gly Lys Leu Ile Gly Asn Ala Ser Ala Asn
   130 135 140 145
   Ala Glu Val Val Asn Asn Cys Val Pro Val Leu Lys Gln Phe Arg Glu
   150 155 160
   Asn Leu Asn Gln Tyr Ala Pro Asp Tyr Val Lys Gly Thr Ala Val Asn
   165 170 175
   Glu Leu Ile Lys Gly Ile Glu Phe Asp Phe Ser Gly Ala Ala Tyr Glu
   180 185 190
   Lys Asp Val Lys Thr Met Pro Trp Tyr Gly Lys Ile Asp Pro Phe Ile
   195 200 205
   Asn Glu Leu Lys Ala Leu Gly Leu Tyr Gly Asn Ile Thr Ser Ala Thr
   210 215 220 225
   Glu Trp Ala Ser Asp Val Gly Ile Tyr Tyr Leu Ser Lys Phe Gly Leu
   230 235 240
   Tyr Ser Thr Asn Arg Asn Asp Ile Val Gln Ser Leu Glu Lys Ala Val
   245 250 255
   Asp Met Tyr Lys Tyr Gly Lys Ile Ala Phe Val Ala Met Glu Arg Ile
   260 265 270
   Thr Trp Asp Tyr Asp Gly Ile Gly Ser Asn Gly Lys Lys Val Asp His
   275 280 285
   Asp Lys Phe Leu Asp Asp Ala Glu Lys His Tyr Leu Pro Lys Thr Tyr
   290 295 300 305
   Thr Phe Asp Asn Gly Thr Phe Ile Ile Arg Ala Gly Asp Lys Val Ser
   310 315 320
   Glu Glu Lys Ile Lys Arg Leu Tyr Trp Ala Ser Arg Glu Val Lys Ser
   325 330 335
   Gln Phe His Arg Val Val Gly Asn Asp Lys Ala Leu Glu Val Gly Asn
   340 345 350
   Ala Asp Asp Val Leu Thr Met Lys Ile Phe Asn Ser Pro Glu Glu Tyr
   355 360 365
   Lys Phe Asn Thr Asn Ile Asn Gly Val Ser Thr Asp Asn Gly Gly Leu
   370 375 380 385
   Tyr Ile Glu Pro Arg Gly Thr Phe Tyr Thr Tyr Glu Arg Thr Pro Gln
   390 395 400
   Gln Ser Ile Phe Ser Leu Glu Glu Leu Phe Arg His Glu Tyr Thr His
   405 410 415
   Tyr Leu Gln Ala Arg Tyr Leu Val Asp Gly Leu Trp Gly Gln Gly Pro
   420 425 430
   Phe Tyr Glu Lys Asn Arg Leu Thr Trp Phe Asp Glu Gly Thr Ala Glu
   435 440 445
   Phe Phe Ala Gly Ser Thr Arg Thr Ser Gly Val Leu Pro Arg Lys Ser
   450 455 460 465
   Ile Leu Gly Tyr Leu Ala Lys Asp Lys Val Asp His Arg Tyr Ser Leu
   470 475 480
   Lys Lys Thr Leu Asn Ser Gly Tyr Asp Asp Ser Asp Trp Met Phe Tyr
   485 490 495
   Asn Tyr Gly Phe Ala Val Ala His Tyr Leu Tyr Glu Lys Asp Met Pro
   500 505 510
   Thr Phe Ile Lys Met Asn Lys Ala Ile Leu Asn Thr Asp Val Lys Ser
   515 520 525
   Tyr Asp Glu Ile Ile Lys Lys Leu Ser Asp Asp Ala Asn Lys Asn Thr
   530 535 540 545
   Glu Tyr Gln Asn His Ile Gln Glu Leu Ala Asp Lys Tyr Gln Gly Ala
   550 555 560
   Gly Ile Pro Leu Val Ser Asp Asp Tyr Leu Lys Asp His Gly Tyr Lys
   565 570 575
   Lys Ala Ser Glu Val Tyr Ser Glu Ile Ser Lys Ala Ala Ser Leu Thr
   580 585 590
   Asn Thr Ser Val Thr Ala Glu Lys Ser Gln Tyr Phe Asn Thr Phe Thr
   595 600 605
   Leu Arg Gly Thr Tyr Thr Gly Glu Thr Ser Lys Gly Glu Phe Lys Asp
   610 615 620 625
   Trp Asp Glu Met Ser Lys Lys Leu Asp Gly Thr Leu Glu Ser Leu Ala
   630 635 640
   Lys Asn Ser Trp Ser Gly Tyr Lys Thr Leu Thr Ala Tyr Phe Thr Asn
   645 650 655
   Tyr Arg Val Thr Ser Asp Asn Lys Val Gln Tyr Asp Val Val Phe His
   660 665 670
   Gly Val Leu Thr Asp Asn Ala Asp Ile Ser Asn Asn Lys Ala Pro Ile
   675 680 685
   Ala Lys Val Thr Gly Pro Ser Thr Gly Ala Val Gly Arg Asn Ile Glu
   690 695 700 705
   Phe Ser Gly Lys Asp Ser Lys Asp Glu Asp Gly Lys Ile Val Ser Tyr
   710 715 720
   Asp Trp Asp Phe Gly Asp Gly Ala Thr Ser Arg Gly Lys Asn Ser Val
   725 730 735
   His Ala Tyr Lys Lys Ala Gly Thr Tyr Asn Val Thr Leu Lys Val Thr
   740 745 750
   Asp Asp Lys Gly Ala Thr Ala Thr Glu Ser Phe Thr Ile Glu Ile Lys
   755 760 765
   Asn Glu Asp Thr Thr Thr Pro Ile Thr Lys Glu Met Glu Pro Asn Asp
   770 775 780 785
   Asp Ile Lys Glu Ala Asn Gly Pro Ile Val Glu Gly Val Thr Val Lys
   790 795 800
   Gly Asp Leu Asn Gly Ser Asp Asp Ala Asp Thr Phe Tyr Phe Asp Val
   805 810 815
   Lys Glu Asp Gly Asp Val Thr Ile Glu Leu Pro Tyr Ser Gly Ser Ser
   820 825 830
   Asn Phe Thr Trp Leu Val Tyr Lys Glu Gly Asp Asp Gln Asn His Ile
   835 840 845
   Ala Ser Gly Ile Asp Lys Asn Asn Ser Lys Val Gly Thr Phe Lys Ser
   850 855 860 865
   Thr Lys Gly Arg His Tyr Val Phe Ile Tyr Lys His Asp Ser Ala Ser
   870 875 880
   Asn Ile Ser Tyr Ser Leu Asn Ile Lys Gly Leu Gly Asn Glu Lys Leu
   885 890 895
   Lys Glu Lys Glu Asn Asn Asp Ser Ser Asp Lys Ala Thr Val Ile Pro
   900 905 910
   Asn Phe Asn Thr Thr Met Gln Gly Ser Leu Leu Gly Asp Asp Ser Arg
   915 920 925
   Asp Tyr Tyr Ser Phe Glu Val Lys Glu Glu Gly Glu Val Asn Ile Glu
   930 935 940 945
   Leu Asp Lys Lys Asp Glu Phe Gly Val Thr Trp Thr Leu His Pro Glu
   950 955 960
   Ser Asn Ile Asn Asp Arg Ile Thr Tyr Gly Gln Val Asp Gly Asn Lys
   965 970 975
   Val Ser Asn Lys Val Lys Leu Arg Pro Gly Lys Tyr Tyr Leu Leu Val
   980 985 990
   Tyr Lys Tyr Ser Gly Ser Gly Asn Tyr Glu Leu Arg Val Ser Arg
   995 1000 1005
<210> 3
   <211> 1131
   <212> PRT
   <213> Clostridium histolyticum
<220>
   <221> SIGNAL
   <222> (-110)..(-1)
<220>
   <221> mat_peptide
   <222> (1)..(1021)
   <223>
<400> 3
   Met Lys Lys Asn Ile Leu Lys Ile Leu Met Asp Ser Tyr Ser Lys
   -110 -105 -100
   Glu Ser Lys Ile Gln Thr Val Arg Arg Val Thr Ser Val Ser Leu Leu
   -95 -90 -85 -80
   Ala Val Tyr Leu Thr Met Asn Thr Ser Ser Leu Val Leu Ala Lys Pro
   -75 -70 -65
   Ile Glu Asn Thr Asn Asp Thr Ser Ile Lys Asn Val Glu Lys Leu Arg
   -60 -55 -50
   Asn Ala Pro Asn Glu Glu Asn Ser Lys Lys Val Glu Asp Ser Lys Asn
   -45 -40 -35
   Asp Lys Val Glu His Val Lys Asn Ile Glu Glu Ala Lys Val Glu Gln
   -30 -25 -20
   Val Ala Pro Glu Val Lys Ser Lys Ser Thr Leu Arg Ser Ala Ser Ile
   -15 -10 -5 -1 1
   Ala Asn Thr Asn Ser Glu Lys Tyr Asp Phe Glu Tyr Leu Asn Gly Leu
   5 10 15
   Ser Tyr Thr Glu Leu Thr Asn Leu Ile Lys Asn Ile Lys Trp Asn Gln
   20 25 30
   Ile Asn Gly Leu Phe Asn Tyr Ser Thr Gly Ser Gln Lys Phe Phe Gly
   35 40 45
   Asp Lys Asn Arg Val Gln Ala Ile Ile Asn Ala Leu Gln Glu Ser Gly
   50 55 60 65
   Arg Thr Tyr Thr Ala Asn Asp Met Lys Gly Ile Glu Thr Phe Thr Glu
   70 75 80
   Val Leu Arg Ala Gly Phe Tyr Leu Gly Tyr Tyr Asn Asp Gly Leu Ser
   85 90 95
   Tyr Leu Asn Asp Arg Asn Phe Gln Asp Lys Cys Ile Pro Ala Met Ile
   100 105 110
   Ala Ile Gln Lys Asn Pro Asn Phe Lys Leu Gly Thr Ala Val Gln Asp
   115 120 125
   Glu Val Ile Thr Ser Leu Gly Lys Leu Ile Gly Asn Ala Ser Ala Asn
   130 135 140 145
   Ala Glu Val Val Asn Asn Cys Val Pro Val Leu Lys Gln Phe Arg Glu
   150 155 160
   Asn Leu Asn Gln Tyr Ala Pro Asp Tyr Val Lys Gly Thr Ala Val Asn
   165 170 175
   Glu Leu Ile Lys Gly Ile Glu Phe Asp Phe Ser Gly Ala Ala Tyr Glu
   180 185 190
   Lys Asp Val Lys Thr Met Pro Trp Tyr Gly Lys Ile Asp Pro Phe Ile
   195 200 205
   Asn Glu Leu Lys Ala Leu Gly Leu Tyr Gly Asn Ile Thr Ser Ala Thr
   210 215 220 225
   Glu Trp Ala Ser Asp Val Gly Ile Tyr Tyr Leu Ser Lys Phe Gly Leu
   230 235 240
   Tyr Ser Thr Asn Arg Asn Asp Ile Val Gln Ser Leu Glu Lys Ala Val
   245 250 255
   Asp Met Tyr Lys Tyr Gly Lys Ile Ala Phe Val Ala Met Glu Arg Ile
   260 265 270
   Thr Trp Asp Tyr Asp Gly Ile Gly Ser Asn Gly Lys Lys Val Asp His
   275 280 285
   Asp Lys Phe Leu Asp Asp Ala Glu Lys His Tyr Leu Pro Lys Thr Tyr
   290 295 300 305
   Thr Phe Asp Asn Gly Thr Phe Ile Ile Arg Ala Gly Asp Lys Val Ser
   310 315 320
   Glu Glu Lys Ile Lys Arg Leu Tyr Trp Ala Ser Arg Glu Val Lys Ser
   325 330 335
   Gln Phe His Arg Val Val Gly Asn Asp Lys Ala Leu Glu Val Gly Asn
   340 345 350
   Ala Asp Asp Val Leu Thr Met Lys Ile Phe Asn Ser Pro Glu Glu Tyr
   355 360 365
   Lys Phe Asn Thr Asn Ile Asn Gly Val Ser Thr Asp Asn Gly Gly Leu
   370 375 380 385
   Tyr Ile Glu Pro Arg Gly Thr Phe Tyr Thr Tyr Glu Arg Thr Pro Gln
   390 395 400
   Gln Ser Ile Phe Ser Leu Glu Glu Leu Phe Arg His Glu Tyr Thr His
   405 410 415
   Tyr Leu Gln Ala Arg Tyr Leu Val Asp Gly Leu Trp Gly Gln Gly Pro
   420 425 430
   Phe Tyr Glu Lys Asn Arg Leu Thr Trp Phe Asp Glu Gly Thr Ala Glu
   435 440 445
   Phe Phe Ala Gly Ser Thr Arg Thr Ser Gly Val Leu Pro Arg Lys Ser
   450 455 460 465
   Ile Leu Gly Tyr Leu Ala Lys Asp Lys Val Asp His Arg Tyr Ser Leu
   470 475 480
   Lys Lys Thr Leu Asn Ser Gly Tyr Asp Asp Ser Asp Trp Met Phe Tyr
   485 490 495
   Asn Tyr Gly Phe Ala Val Ala His Tyr Leu Tyr Glu Lys Asp Met Pro
   500 505 510
   Thr Phe Ile Lys Met Asn Lys Ala Ile Leu Asn Thr Asp Val Lys Ser
   515 520 525
   Tyr Asp Glu Ile Ile Lys Lys Leu Ser Asp Asp Ala Asn Lys Asn Thr
   530 535 540 545
   Glu Tyr Gln Asn His Ile Gln Glu Leu Ala Asp Lys Tyr Gln Gly Ala
   550 555 560
   Gly Ile Pro Leu Val Ser Asp Asp Tyr Leu Lys Asp His Gly Tyr Lys
   565 570 575
   Lys Ala Ser Glu Val Tyr Ser Glu Ile Ser Lys Ala Ala Ser Leu Thr
   580 585 590
   Asn Thr Ser Val Thr Ala Glu Lys Ser Gln Tyr Phe Asn Thr Phe Thr
   595 600 605
   Leu Arg Gly Thr Tyr Thr Gly Glu Thr Ser Lys Gly Glu Phe Lys Asp
   610 615 620 625
   Trp Asp Glu Met Ser Lys Lys Leu Asp Gly Thr Leu Glu Ser Leu Ala
   630 635 640
   Lys Asn Ser Trp Ser Gly Tyr Lys Thr Leu Thr Ala Tyr Phe Thr Asn
   645 650 655
   Tyr Arg Val Thr Ser Asp Asn Lys Val Gln Tyr Asp Val Val Phe His
   660 665 670
   Gly Val Leu Thr Asp Asn Ala Asp Ile Ser Asn Asn Lys Ala Pro Ile
   675 680 685
   Ala Lys Val Thr Gly Pro Ser Thr Gly Ala Val Gly Arg Asn Ile Glu
   690 695 700 705
   Phe Ser Gly Lys Asp Ser Lys Asp Glu Asp Gly Lys Ile Val Ser Tyr
   710 715 720
   Asp Trp Asp Phe Gly Asp Gly Ala Thr Ser Arg Gly Lys Asn Ser Val
   725 730 735
   His Ala Tyr Lys Lys Ala Gly Thr Tyr Asn Val Thr Leu Lys Val Thr
   740 745 750
   Asp Asp Lys Gly Ala Thr Ala Thr Glu Ser Phe Thr Ile Glu Ile Lys
   755 760 765
   Asn Glu Asp Thr Thr Thr Pro Ile Thr Lys Glu Met Glu Pro Asn Asp
   770 775 780 785
   Asp Ile Lys Glu Ala Asn Gly Pro Ile Val Glu Gly Val Thr Val Lys
   790 795 800
   Gly Asp Leu Asn Gly Ser Asp Asp Ala Asp Thr Phe Tyr Phe Asp Val
   805 810 815
   Lys Glu Asp Gly Asp Val Thr Ile Glu Leu Pro Tyr Ser Gly Ser Ser
   820 825 830
   Asn Phe Thr Trp Leu Val Tyr Lys Glu Gly Asp Asp Gln Asn His Ile
   835 840 845
   Ala Ser Gly Ile Asp Lys Asn Asn Ser Lys Val Gly Thr Phe Lys Ser
   850 855 860 865
   Thr Lys Gly Arg His Tyr Val Phe Ile Tyr Lys His Asp Ser Ala Ser
   870 875 880
   Asn Ile Ser Tyr Ser Leu Asn Ile Lys Gly Leu Gly Asn Glu Lys Leu
   885 890 895
   Lys Glu Lys Glu Asn Asn Asp Ser Ser Asp Lys Ala Thr Val Ile Pro
   900 905 910
   Asn Phe Asn Thr Thr Met Gln Gly Ser Leu Leu Gly Asp Asp Ser Arg
   915 920 925
   Asp Tyr Tyr Ser Phe Glu Val Lys Glu Glu Gly Glu Val Asn Ile Glu
   930 935 940 945
   Leu Asp Lys Lys Asp Glu Phe Gly Val Thr Trp Thr Leu His Pro Glu
   950 955 960
   Ser Asn Ile Asn Asp Arg Ile Thr Tyr Gly Gln Val Asp Gly Asn Lys
   965 970 975
   Val Ser Asn Lys Val Lys Leu Arg Pro Gly Lys Tyr Tyr Leu Leu Val
   980 985 990
   Tyr Lys Tyr Ser Gly Ser Gly Asn Tyr Glu Leu Arg Val Ser Arg Lys
   995 1000 1005
   Leu Ala Ala Ala Leu Glu His His His His His His
   1010 1015 1020
<210> 4
   <211> 3396
   <212> DNA
   <213> Clostridium histolyticum
<220>
   <221> CDS
   <222> (1)..(3396)
<400> 4
   atg aaa aaa aat att tta aag att ctt atg gat agt tat tct aaa gaa 48
   Met Lys Lys Asn Ile Leu Lys Ile Leu Met Asp Ser Tyr Ser Lys Glu
   1 5 10 15
   tct aaa att caa act gta cgt agg gtt acg agt gta tca ctt tta gcg 96
   Ser Lys Ile Gln Thr Val Arg Arg Val Thr Ser Val Ser Leu Leu Ala
   20 25 30
   gta tat ctt act atg aat act tca agt tta gtt tta gca aaa cca ata 144
   Val Tyr Leu Thr Met Asn Thr Ser Ser Leu Val Leu Ala Lys Pro Ile
   35 40 45
   gaa aat act aat gat act agt ata aaa aat gtg gag aaa tta aga aat 192
   Glu Asn Thr Asn Asp Thr Ser Ile Lys Asn Val Glu Lys Leu Arg Asn
   50 55 60
   gct cca aat gaa gag aat agt aaa aag gta gaa gat agt aaa aat gat 240
   Ala Pro Asn Glu Glu Asn Ser Lys Lys Val Glu Asp Ser Lys Asn Asp
   65 70 75 80
   aag gta gaa cat gtg aaa aat ata gaa gag gca aag gtt gag caa gtt 288
   Lys Val Glu His Val Lys Asn Ile Glu Glu Ala Lys Val Glu Gln Val
   85 90 95
   gca ccc gaa gta aaa tct aaa tca act tta aga agt gct tct ata gcg 336
   Ala Pro Glu Val Lys Ser Lys Ser Thr Leu Arg Ser Ala Ser Ile Ala
   100 105 110
   aat act aat tct gag aaa tat gat ttt gag tat tta aat ggt ttg agc 384
   Asn Thr Asn Ser Glu Lys Tyr Asp Phe Glu Tyr Leu Asn Gly Leu Ser
   115 120 125
   tat act gaa ctt aca aat tta att aaa aat ata aag tgg aat caa att 432
   Tyr Thr Glu Leu Thr Asn Leu Ile Lys Asn Ile Lys Trp Asn Gln Ile
   130 135 140
   aat ggt tta ttt aat tat agt aca ggt tct caa aag ttc ttt gga gat 480
   Asn Gly Leu Phe Asn Tyr Ser Thr Gly Ser Gln Lys Phe Phe Gly Asp
   145 150 155 160
   aaa aat cgt gta caa gct ata att aat gct tta caa gaa agt gga aga 528
   Lys Asn Arg Val Gln Ala Ile Ile Asn Ala Leu Gln Glu Ser Gly Arg
   165 170 175
   act tac act gca aat gat atg aag ggt ata gaa act ttc act gag gtt 576
   Thr Tyr Thr Ala Asn Asp Met Lys Gly Ile Glu Thr Phe Thr Glu Val
   180 185 190
   tta aga gct ggt ttt tat tta ggg tac tat aat gat ggt tta tct tat 624
   Leu Arg Ala Gly Phe Tyr Leu Gly Tyr Tyr Asn Asp Gly Leu Ser Tyr
   195 200 205
   tta aat gat aga aac ttc caa gat aaa tgt ata cct gca atg att gca 672
   Leu Asn Asp Arg Asn Phe Gln Asp Lys Cys Ile Pro Ala Met Ile Ala
   210 215 220
   att caa aaa aat cct aac ttt aag cta gga act gca gtt caa gat gaa 720
   Ile Gln Lys Asn Pro Asn Phe Lys Leu Gly Thr Ala Val Gln Asp Glu
   225 230 235 240
   gtt ata act tct tta gga aaa cta ata gga aat gct tct gct aat gct 768
   Val Ile Thr Ser Leu Gly Lys Leu Ile Gly Asn Ala Ser Ala Asn Ala
   245 250 255
   gaa gta gtt aat aat tgt gta cca gtt cta aaa caa ttt aga gaa aac 816
   Glu Val Val Asn Asn Cys Val Pro Val Leu Lys Gln Phe Arg Glu Asn
   260 265 270
   tta aat caa tat gct cct gat tac gtt aaa gga aca gct gta aat gaa 864
   Leu Asn Gln Tyr Ala Pro Asp Tyr Val Lys Gly Thr Ala Val Asn Glu
   275 280 285
   tta att aaa ggt att gaa ttc gat ttt tct ggt gct gca tat gaa aaa 912
   Leu Ile Lys Gly Ile Glu Phe Asp Phe Ser Gly Ala Ala Tyr Glu Lys
   290 295 300
   gat gtt aag aca atg cct tgg tat gga aaa att gat cca ttt ata aat 960
   Asp Val Lys Thr Met Pro Trp Tyr Gly Lys Ile Asp Pro Phe Ile Asn
   305 310 315 320
   gaa ctt aag gcc tta ggt cta tat gga aat ata aca agt gca act gag 1008
   Glu Leu Lys Ala Leu Gly Leu Tyr Gly Asn Ile Thr Ser Ala Thr Glu
   325 330 335
   tgg gca tct gat gtt gga ata tac tat tta agt aaa ttc ggt ctt tac 1056
   Trp Ala Ser Asp Val Gly Ile Tyr Tyr Leu Ser Lys Phe Gly Leu Tyr
   340 345 350
   tca act aac cga aat gac ata gta cag tca ctt gaa aag gct gta gat 1104
   Ser Thr Asn Arg Asn Asp Ile Val Gln Ser Leu Glu Lys Ala Val Asp
   355 360 365
   atg tat aag tat ggt aaa ata gcc ttt gta gca atg gag aga ata act 1152
   Met Tyr Lys Tyr Gly Lys Ile Ala Phe Val Ala Met Glu Arg Ile Thr
   370 375 380
   tgg gat tat gat ggg att ggt tct aat ggt aaa aag gtg gat cac gat 1200
   Trp Asp Tyr Asp Gly Ile Gly Ser Asn Gly Lys Lys Val Asp His Asp
   385 390 395 400
   aag ttc tta gat gat gct gaa aaa cat tat ctg cca aag aca tat act 1248
   Lys Phe Leu Asp Asp Ala Glu Lys His Tyr Leu Pro Lys Thr Tyr Thr
   405 410 415
   ttt gat aat gga acc ttt att ata aga gca ggg gat aag gta tcc gaa 1296
   Phe Asp Asn Gly Thr Phe Ile Ile Arg Ala Gly Asp Lys Val Ser Glu
   420 425 430
   gaa aaa ata aaa agg cta tat tgg gca tca aga gaa gtg aag tct caa 1344
   Glu Lys Ile Lys Arg Leu Tyr Trp Ala Ser Arg Glu Val Lys Ser Gln
   435 440 445
   ttc cat aga gta gtt ggc aat gat aaa gct tta gag gtg gga aat gcc 1392
   Phe His Arg Val Val Gly Asn Asp Lys Ala Leu Glu Val Gly Asn Ala
   450 455 460
   gat gat gtt tta act atg aaa ata ttt aat agc cca gaa gaa tat aaa 1440
   Asp Asp Val Leu Thr Met Lys Ile Phe Asn Ser Pro Glu Glu Tyr Lys
   465 470 475 480
   ttt aat acc aat ata aat ggt gta agc act gat aat ggt ggt cta tat 1488
   Phe Asn Thr Asn Ile Asn Gly Val Ser Thr Asp Asn Gly Gly Leu Tyr
   485 490 495
   ata gaa cca aga ggg act ttc tac act tat gag aga aca cct caa caa 1536
   Ile Glu Pro Arg Gly Thr Phe Tyr Thr Tyr Glu Arg Thr Pro Gln Gln
   500 505 510
   agt ata ttt agt ctt gaa gaa ttg ttt aga cat gaa tat act cac tat 1584
   Ser Ile Phe Ser Leu Glu Glu Leu Phe Arg His Glu Tyr Thr His Tyr
   515 520 525
   tta caa gcg aga tat ctt gta gat ggt tta tgg ggg caa ggt cca ttt 1632
   Leu Gln Ala Arg Tyr Leu Val Asp Gly Leu Trp Gly Gln Gly Pro Phe
   530 535 540
   tat gaa aaa aat aga tta act tgg ttt gat gaa ggt aca gct gaa ttc 1680
   Tyr Glu Lys Asn Arg Leu Thr Trp Phe Asp Glu Gly Thr Ala Glu Phe
   545 550 555 560
   ttt gca gga tct acc cgt aca tct ggt gtt tta cca aga aaa tca ata 1728
   Phe Ala Gly Ser Thr Arg Thr Ser Gly Val Leu Pro Arg Lys Ser Ile
   565 570 575
   tta gga tat ttg gct aag gat aaa gta gat cat aga tac tca tta aag 1776
   Leu Gly Tyr Leu Ala Lys Asp Lys Val Asp His Arg Tyr Ser Leu Lys
   580 585 590
   aag act ctt aat tca ggg tat gat gac agt gat tgg atg ttc tat aat 1824
   Lys Thr Leu Asn Ser Gly Tyr Asp Asp Ser Asp Trp Met Phe Tyr Asn
   595 600 605
   tat gga ttt gca gtt gca cat tac cta tat gaa aaa gat atg cct aca 1872
   Tyr Gly Phe Ala Val Ala His Tyr Leu Tyr Glu Lys Asp Met Pro Thr
   610 615 620
   ttt att aag atg aat aaa gct ata ttg aat aca gat gtg aaa tct tat 1920
   Phe Ile Lys Met Asn Lys Ala Ile Leu Asn Thr Asp Val Lys Ser Tyr
   625 630 635 640
   gat gaa ata ata aaa aaa tta agt gat gat gca aat aaa aat aca gaa 1968
   Asp Glu Ile Ile Lys Lys Leu Ser Asp Asp Ala Asn Lys Asn Thr Glu
   645 650 655
   tat caa aac cat att caa gag tta gca gat aaa tat caa gga gca ggc 2016
   Tyr Gln Asn His Ile Gln Glu Leu Ala Asp Lys Tyr Gln Gly Ala Gly
   660 665 670
   ata cct cta gta tca gat gat tac tta aaa gat cat gga tat aag aaa 2064
   Ile Pro Leu Val Ser Asp Asp Tyr Leu Lys Asp His Gly Tyr Lys Lys
   675 680 685
   gca tct gaa gta tat tct gaa att tca aaa gct gct tct ctt aca aac 2112
   Ala Ser Glu Val Tyr Ser Glu Ile Ser Lys Ala Ala Ser Leu Thr Asn
   690 695 700
   act agt gta aca gca gaa aaa tct caa tat ttt aac aca ttc act tta 2160
   Thr Ser Val Thr Ala Glu Lys Ser Gln Tyr Phe Asn Thr Phe Thr Leu
   705 710 715 720
   aga gga act tat aca ggt gaa act tct aaa ggt gaa ttt aaa gat tgg 2208
   Arg Gly Thr Tyr Thr Gly Glu Thr Ser Lys Gly Glu Phe Lys Asp Trp
   725 730 735
   gat gaa atg agt aaa aaa tta gat gga act ttg gag tcc ctt gct aaa 2256
   Asp Glu Met Ser Lys Lys Leu Asp Gly Thr Leu Glu Ser Leu Ala Lys
   740 745 750
   aat tct tgg agt gga tac aaa act tta aca gca tac ttt acg aat tat 2304
   Asn Ser Trp Ser Gly Tyr Lys Thr Leu Thr Ala Tyr Phe Thr Asn Tyr
   755 760 765
   aga gtt aca agc gat aat aaa gtt caa tat gat gta gtt ttc cat ggg 2352
   Arg Val Thr Ser Asp Asn Lys Val Gln Tyr Asp Val Val Phe His Gly
   770 775 780
   gtt tta aca gat aat gcg gat att agt aac aat aag gct cca ata gca 2400
   Val Leu Thr Asp Asn Ala Asp Ile Ser Asn Asn Lys Ala Pro Ile Ala
   785 790 795 800
   aag gta act gga cca agc act ggt gct gta gga aga aat att gaa ttt 2448
   Lys Val Thr Gly Pro Ser Thr Gly Ala Val Gly Arg Asn Ile Glu Phe
   805 810 815
   agt gga aaa gat agt aaa gat gaa gat ggt aaa ata gta tca tat gat 2496
   Ser Gly Lys Asp Ser Lys Asp Glu Asp Gly Lys Ile Val Ser Tyr Asp
   820 825 830
   tgg gat ttt ggc gat ggt gca act agt aga ggc aaa aat tca gta cat 2544
   Trp Asp Phe Gly Asp Gly Ala Thr Ser Arg Gly Lys Asn Ser Val His
   835 840 845
   gct tac aaa aaa gca gga aca tat aat gtt aca tta aaa gta act gac 2592
   Ala Tyr Lys Lys Ala Gly Thr Tyr Asn Val Thr Leu Lys Val Thr Asp
   850 855 860
   gat aag ggt gca aca gct aca gaa agc ttt act ata gaa ata aag aac 2640
   Asp Lys Gly Ala Thr Ala Thr Glu Ser Phe Thr Ile Glu Ile Lys Asn
   865 870 875 880
   gaa gat aca aca aca cct ata act aaa gaa atg gaa cct aat gat gat 2688
   Glu Asp Thr Thr Thr Pro Ile Thr Lys Glu Met Glu Pro Asn Asp Asp
   885 890 895
   ata aaa gag gct aat ggt cca ata gtt gaa ggt gtt act gta aaa ggt 2736
   Ile Lys Glu Ala Asn Gly Pro Ile Val Glu Gly Val Thr Val Lys Gly
   900 905 910
   gat tta aat ggt tct gat gat gct gat acc ttc tat ttt gat gta aaa 2784
   Asp Leu Asn Gly Ser Asp Asp Ala Asp Thr Phe Tyr Phe Asp Val Lys
   915 920 925
   gaa gat ggt gat gtt aca att gaa ctt cct tat tca ggg tca tct aat 2832
   Glu Asp Gly Asp Val Thr Ile Glu Leu Pro Tyr Ser Gly Ser Ser Asn
   930 935 940
   ttc aca tgg tta gtt tat aaa gag gga gac gat caa aac cat att gca 2880
   Phe Thr Trp Leu Val Tyr Lys Glu Gly Asp Asp Gln Asn His Ile Ala
   945 950 955 960
   agt ggt ata gat aag aat aac tca aaa gtt gga aca ttt aaa tct aca 2928
   Ser Gly Ile Asp Lys Asn Asn Ser Lys Val Gly Thr Phe Lys Ser Thr
   965 970 975
   aaa gga aga cat tat gtg ttt ata tat aaa cac gat tct gct tca aat 2976
   Lys Gly Arg His Tyr Val Phe Ile Tyr Lys His Asp Ser Ala Ser Asn
   980 985 990
   ata tcc tat tct tta aac ata aaa gga tta ggt aac gag aaa ttg aag 3024
   Ile Ser Tyr Ser Leu Asn Ile Lys Gly Leu Gly Asn Glu Lys Leu Lys
   995 1000 1005
   gaa aaa gaa aat aat gat tct tct gat aaa gct aca gtt ata cca 3069
   Glu Lys Glu Asn Asn Asp Ser Ser Asp Lys Ala Thr Val Ile Pro
   1010 1015 1020
   aat ttc aat acc act atg caa ggt tca ctt tta ggt gat gat tca 3114
   Asn Phe Asn Thr Thr Met Gln Gly Ser Leu Leu Gly Asp Asp Ser
   1025 1030 1035
   aga gat tat tat tct ttt gag gtt aag gaa gaa ggc gaa gtt aat 3159
   Arg Asp Tyr Tyr Ser Phe Glu Val Lys Glu Glu Gly Glu Val Asn
   1040 1045 1050
   ata gaa cta gat aaa aag gat gaa ttt ggt gta aca tgg aca cta 3204
   Ile Glu Leu Asp Lys Lys Asp Glu Phe Gly Val Thr Trp Thr Leu
   1055 1060 1065
   cat cca gag tca aat att aat gac aga ata act tac gga caa gtt 3249
   His Pro Glu Ser Asn Ile Asn Asp Arg Ile Thr Tyr Gly Gln Val
   1070 1075 1080
   gat ggt aat aag gta tct aat aaa gtt aaa tta aga cca gga aaa 3294
   Asp Gly Asn Lys Val Ser Asn Lys Val Lys Leu Arg Pro Gly Lys
   1085 1090 1095
   tat tat cta ctt gtt tat aaa tac tca gga tca gga aac tat gag 3339
   Tyr Tyr Leu Leu Val Tyr Lys Tyr Ser Gly Ser Gly Asn Tyr Glu
   1100 1105 1110
   tta agg gta tct aga aag ctt gcg gcc gca ctc gag cac cac cac 3384
   Leu Arg Val Ser Arg Lys Leu Ala Ala Ala Leu Glu His His His
   1115 1120 1125
   cac cac cac tga 3396
   His His His
   1130
<210> 5
   <211> 1021
   <212> PRT
   <213> Clostridium histolyticum
<220>
   <221> SIGNAL
   <222> (-40)..(-1)
<220>
   <221> mat_peptide
   <222> (1)..(981)
<400> 5
   Met Lys Arg Lys Cys Leu Ser Lys Arg Leu Met Leu Ala Ile Thr Met
   -40 -35 -30 -25
   Ala Thr Ile Phe Thr Val Asn Ser Thr Leu Pro Ile Tyr Ala Ala Val
   -20 -15 -10
   Asp Lys Asn Asn Ala Thr Ala Ala Val Gln Asn Glu Ser Lys Arg Tyr
   -5 -1 1 5
   Thr Val Ser Tyr Leu Lys Thr Leu Asn Tyr Tyr Asp Leu Val Asp Leu
   10 15 20
   Leu Val Lys Thr Glu Ile Glu Asn Leu Pro Asp Leu Phe Gln Tyr Ser
   25 30 35 40
   Ser Asp Ala Lys Glu Phe Tyr Gly Asn Lys Thr Arg Met Ser Phe Ile
   45 50 55
   Met Asp Glu Ile Gly Arg Arg Ala Pro Gln Tyr Thr Glu Ile Asp His
   60 65 70
   Lys Gly Ile Pro Thr Leu Val Glu Val Val Arg Ala Gly Phe Tyr Leu
   75 80 85
   Gly Phe His Asn Lys Glu Leu Asn Glu Ile Asn Lys Arg Ser Phe Lys
   90 95 100
   Glu Arg Val Ile Pro Ser Ile Leu Ala Ile Gln Lys Asn Pro Asn Phe
   105 110 115 120
   Lys Leu Gly Thr Glu Val Gln Asp Lys Ile Val Ser Ala Thr Gly Leu
   125 130 135
   Leu Ala Gly Asn Glu Thr Ala Pro Pro Glu Val Val Asn Asn Phe Thr
   140 145 150
   Pro Ile Leu Gln Asp Cys Ile Lys Asn Ile Asp Arg Tyr Ala Leu Asp
   155 160 165
   Asp Leu Lys Ser Lys Ala Leu Phe Asn Val Leu Ala Ala Pro Thr Tyr
   170 175 180
   Asp Ile Thr Glu Tyr Leu Arg Ala Thr Lys Glu Lys Pro Glu Asn Thr
   185 190 195 200
   Pro Trp Tyr Gly Lys Ile Asp Gly Phe Ile Asn Glu Leu Lys Lys Leu
   205 210 215
   Ala Leu Tyr Gly Lys Ile Asn Asp Asn Asn Ser Trp Ile Ile Asp Asn
   220 225 230
   Gly Ile Tyr His Ile Ala Pro Leu Gly Lys Leu His Ser Asn Asn Lys
   235 240 245
   Ile Gly Ile Glu Thr Leu Thr Glu Val Met Lys Val Tyr Pro Tyr Leu
   250 255 260
   Ser Met Gln His Leu Gln Ser Ala Asp Gln Ile Lys Arg His Tyr Asp
   265 270 275 280
   Ser Lys Asp Ala Glu Gly Asn Lys Ile Pro Leu Asp Lys Phe Lys Lys
   285 290 295
   Glu Gly Lys Glu Lys Tyr Cys Pro Lys Thr Tyr Thr Phe Asp Asp Gly
   300 305 310
   Lys Val Ile Ile Lys Ala Gly Ala Arg Val Glu Glu Glu Lys Val Lys
   315 320 325
   Arg Leu Tyr Trp Ala Ser Lys Glu Val Asn Ser Gln Phe Phe Arg Val
   330 335 340
   Tyr Gly Ile Asp Lys Pro Leu Glu Glu Gly Asn Pro Asp Asp Ile Leu
   345 350 355 360
   Thr Met Val Ile Tyr Asn Ser Pro Glu Glu Tyr Lys Leu Asn Ser Val
   365 370 375
   Leu Tyr Gly Tyr Asp Thr Asn Asn Gly Gly Met Tyr Ile Glu Pro Glu
   380 385 390
   Gly Thr Phe Phe Thr Tyr Glu Arg Glu Ala Gln Glu Ser Thr Tyr Thr
   395 400 405
   Leu Glu Glu Leu Phe Arg His Glu Tyr Thr His Tyr Leu Gln Gly Arg
   410 415 420
   Tyr Ala Val Pro Gly Gln Trp Gly Arg Thr Lys Leu Tyr Asp Asn Asp
   425 430 435 440
   Arg Leu Thr Trp Tyr Glu Glu Gly Gly Ala Glu Leu Phe Ala Gly Ser
   445 450 455
   Thr Arg Thr Ser Gly Ile Leu Pro Arg Lys Ser Ile Val Ser Asn Ile
   460 465 470
   His Asn Thr Thr Arg Asn Asn Arg Tyr Lys Leu Ser Asp Thr Val His
   475 480 485
   Ser Lys Tyr Gly Ala Ser Phe Glu Phe Tyr Asn Tyr Ala Cys Met Phe
   490 495 500
   Met Asp Tyr Met Tyr Asn Lys Asp Met Gly Ile Leu Asn Lys Leu Asn
   505 510 515 520
   Asp Leu Ala Lys Asn Asn Asp Val Asp Gly Tyr Asp Asn Tyr Ile Arg
   525 530 535
   Asp Leu Ser Ser Asn Tyr Ala Leu Asn Asp Lys Tyr Gln Asp His Met
   540 545 550
   Gln Glu Arg Ile Asp Asn Tyr Glu Asn Leu Thr Val Pro Phe Val Ala
   555 560 565
   Asp Asp Tyr Leu Val Arg His Ala Tyr Lys Asn Pro Asn Glu Ile Tyr
   570 575 580
   Ser Glu Ile Ser Glu Val Ala Lys Leu Lys Asp Ala Lys Ser Glu Val
   585 590 595 600
   Lys Lys Ser Gln Tyr Phe Ser Thr Phe Thr Leu Arg Gly Ser Tyr Thr
   605 610 615
   Gly Gly Ala Ser Lys Gly Lys Leu Glu Asp Gln Lys Ala Met Asn Lys
   620 625 630
   Phe Ile Asp Asp Ser Leu Lys Lys Leu Asp Thr Tyr Ser Trp Ser Gly
   635 640 645
   Tyr Lys Thr Leu Thr Ala Tyr Phe Thr Asn Tyr Lys Val Asp Ser Ser
   650 655 660
   Asn Arg Val Thr Tyr Asp Val Val Phe His Gly Tyr Leu Pro Asn Glu
   665 670 675 680
   Gly Asp Ser Lys Asn Ser Leu Pro Tyr Gly Lys Ile Asn Gly Thr Tyr
   685 690 695
   Lys Gly Thr Glu Lys Glu Lys Ile Lys Phe Ser Ser Glu Gly Ser Phe
   700 705 710
   Asp Pro Asp Gly Lys Ile Val Ser Tyr Glu Trp Asp Phe Gly Asp Gly
   715 720 725
   Asn Lys Ser Asn Glu Glu Asn Pro Glu His Ser Tyr Asp Lys Val Gly
   730 735 740
   Thr Tyr Thr Val Lys Leu Lys Val Thr Asp Asp Lys Gly Glu Ser Ser
   745 750 755 760
   Val Ser Thr Thr Thr Ala Glu Ile Lys Asp Leu Ser Glu Asn Lys Leu
   765 770 775
   Pro Val Ile Tyr Met His Val Pro Lys Ser Gly Ala Leu Asn Gln Lys
   780 785 790
   Val Val Phe Tyr Gly Lys Gly Thr Tyr Asp Pro Asp Gly Ser Ile Ala
   795 800 805
   Gly Tyr Gln Trp Asp Phe Gly Asp Gly Ser Asp Phe Ser Ser Glu Gln
   810 815 820
   Asn Pro Ser His Val Tyr Thr Lys Lys Gly Glu Tyr Thr Val Thr Leu
   825 830 835 840
   Arg Val Met Asp Ser Ser Gly Gln Met Ser Glu Lys Thr Met Lys Ile
   845 850 855
   Lys Ile Thr Asp Pro Val Tyr Pro Ile Gly Thr Glu Lys Glu Pro Asn
   860 865 870
   Asn Ser Lys Glu Thr Ala Ser Gly Pro Ile Val Pro Gly Ile Pro Val
   875 880 885
   Ser Gly Thr Ile Glu Asn Thr Ser Asp Gln Asp Tyr Phe Tyr Phe Asp
   890 895 900
   Val Ile Thr Pro Gly Glu Val Lys Ile Asp Ile Asn Lys Leu Gly Tyr
   905 910 915 920
   Gly Gly Ala Thr Trp Val Val Tyr Asp Glu Asn Asn Asn Ala Val Ser
   925 930 935
   Tyr Ala Thr Asp Asp Gly Gln Asn Leu Ser Gly Lys Phe Lys Ala Asp
   940 945 950
   Lys Pro Gly Arg Tyr Tyr Ile His Leu Tyr Met Phe Asn Gly Ser Tyr
   955 960 965
   Met Pro Tyr Arg Ile Asn Ile Glu Gly Ser Val Gly Arg
   970 975 980
<210> 6
   <211> 1021
   <212> PRT
   <213> Clostridium histolyticum
<220>
   <221> SIGNAL
   <222> (-40)..(-1)
<220>
   <221> mat_peptide
   <222> (1)..(981)
<400> 6
   Met Lys Arg Lys Cys Leu Ser Lys Arg Leu Met Leu Ala Ile Thr Met
   -40 -35 -30 -25
   Ala Thr Ile Phe Thr Val Asn Ser Thr Leu Pro Ile Tyr Ala Ala Val
   -20 -15 -10
   Asp Lys Asn Asn Ala Thr Ala Ala Val Gln Asn Glu Ser Lys Arg Tyr
   -5 -1 1 5
   Thr Val Ser Tyr Leu Lys Thr Leu Asn Tyr Tyr Asp Leu Val Asp Leu
   10 15 20
   Leu Val Lys Thr Glu Ile Glu Asn Leu Pro Asp Leu Phe Gln Tyr Ser
   25 30 35 40
   Ser Asp Ala Lys Glu Phe Tyr Gly Asn Lys Thr Arg Met Ser Phe Ile
   45 50 55
   Met Asp Glu Ile Gly Arg Arg Ala Pro Gln Tyr Thr Glu Ile Asp His
   60 65 70
   Lys Gly Ile Pro Thr Leu Val Glu Val Val Arg Ala Gly Phe Tyr Leu
   75 80 85
   Gly Phe His Asn Lys Glu Leu Asn Glu Ile Asn Lys Arg Ser Phe Lys
   90 95 100
   Glu Arg Val Ile Pro Ser Ile Leu Ala Ile Gln Lys Asn Pro Asn Phe
   105 110 115 120
   Lys Leu Gly Thr Glu Val Gln Asp Lys Ile Val Ser Ala Thr Gly Leu
   125 130 135
   Leu Ala Gly Asn Glu Thr Ala Pro Pro Glu Val Val Asn Asn Phe Thr
   140 145 150
   Pro Ile Leu Gln Asp Cys Ile Lys Asn Ile Asp Arg Tyr Ala Leu Asp
   155 160 165
   Asp Leu Lys Ser Lys Ala Leu Phe Asn Val Leu Ala Ala Pro Thr Tyr
   170 175 180
   Asp Ile Thr Glu Tyr Leu Arg Ala Thr Lys Glu Lys Pro Glu Asn Thr
   185 190 195 200
   Pro Trp Tyr Gly Lys Ile Asp Gly Phe Ile Asn Glu Leu Lys Lys Leu
   205 210 215
   Ala Leu Tyr Gly Lys Ile Asn Asp Asn Asn Ser Trp Ile Ile Asp Asn
   220 225 230
   Gly Ile Tyr His Ile Ala Pro Leu Gly Lys Leu His Ser Asn Asn Lys
   235 240 245
   Ile Gly Ile Glu Thr Leu Thr Glu Val Met Lys Val Tyr Pro Tyr Leu
   250 255 260
   Ser Met Gln His Leu Gln Ser Ala Asp Gln Ile Lys Arg His Tyr Asp
   265 270 275 280
   Ser Lys Asp Ala Glu Gly Asn Lys Ile Pro Leu Asp Lys Phe Lys Lys
   285 290 295
   Glu Gly Lys Glu Lys Tyr Cys Pro Lys Thr Tyr Thr Phe Asp Asp Gly
   300 305 310
   Lys Val Ile Ile Lys Ala Gly Ala Arg Val Glu Glu Glu Lys Val Lys
   315 320 325
   Arg Leu Tyr Trp Ala Ser Lys Glu Val Asn Ser Gln Phe Phe Arg Val
   330 335 340
   Tyr Gly Ile Asp Lys Pro Leu Glu Glu Gly Asn Pro Asp Asp Ile Leu
   345 350 355 360
   Thr Met Val Ile Tyr Asn Ser Pro Glu Glu Tyr Lys Leu Asn Ser Val
   365 370 375
   Leu Tyr Gly Tyr Asp Thr Asn Asn Gly Gly Met Tyr Ile Glu Pro Glu
   380 385 390
   Gly Thr Phe Phe Thr Tyr Glu Arg Glu Ala Gln Glu Ser Thr Tyr Thr
   395 400 405
   Leu Glu Glu Leu Phe Arg His Glu Tyr Thr His Tyr Leu Gln Gly Arg
   410 415 420
   Tyr Ala Val Pro Gly Gln Trp Gly Arg Thr Lys Leu Tyr Asp Asn Asp
   425 430 435 440
   Arg Leu Thr Trp Tyr Glu Glu Gly Gly Ala Glu Leu Phe Ala Gly Ser
   445 450 455
   Thr Arg Thr Ser Gly Ile Leu Pro Arg Lys Ser Ile Val Ser Asn Ile
   460 465 470
   His Asn Thr Thr Arg Asn Asn Arg Tyr Lys Leu Ser Asp Thr Val His
   475 480 485
   Ser Lys Tyr Gly Ala Ser Phe Glu Phe Tyr Asn Tyr Ala Cys Met Phe
   490 495 500
   Met Asp Tyr Met Tyr Asn Lys Asp Met Gly Ile Leu Asn Lys Leu Asn
   505 510 515 520
   Asp Leu Ala Lys Asn Asn Asp Val Asp Gly Tyr Asp Asn Tyr Ile Arg
   525 530 535
   Asp Leu Ser Ser Asn Tyr Ala Leu Asn Asp Lys Tyr Gln Asp His Met
   540 545 550
   Gln Glu Arg Ile Asp Asn Tyr Glu Asn Leu Thr Val Pro Phe Val Ala
   555 560 565
   Asp Asp Tyr Leu Val Arg His Ala Tyr Lys Asn Pro Asn Glu Ile Tyr
   570 575 580
   Ser Glu Ile Ser Glu Val Ala Lys Leu Lys Asp Ala Lys Ser Glu Val
   585 590 595 600
   Lys Lys Ser Gln Tyr Phe Ser Thr Phe Thr Leu Arg Gly Ser Tyr Thr
   605 610 615
   Gly Gly Ala Ser Lys Gly Lys Leu Glu Asp Gln Lys Ala Met Asn Lys
   620 625 630
   Phe Ile Asp Asp Ser Leu Lys Lys Leu Asp Thr Tyr Ser Trp Ser Gly
   635 640 645
   Tyr Lys Thr Leu Thr Ala Tyr Phe Thr Asn Tyr Lys Val Asp Ser Ser
   650 655 660
   Asn Arg Val Thr Tyr Asp Val Val Phe His Gly Tyr Leu Pro Asn Glu
   665 670 675 680
   Gly Asp Ser Lys Asn Ser Leu Pro Tyr Gly Lys Ile Asn Gly Thr Tyr
   685 690 695
   Lys Gly Thr Glu Lys Glu Lys Ile Lys Phe Ser Ser Glu Gly Ser Phe
   700 705 710
   Asp Pro Asp Gly Lys Ile Val Ser Tyr Glu Trp Asp Phe Gly Asp Gly
   715 720 725
   Asn Lys Ser Asn Glu Glu Asn Pro Glu His Ser Tyr Asp Lys Val Gly
   730 735 740
   Thr Tyr Thr Val Lys Leu Lys Val Thr Asp Asp Lys Gly Glu Ser Ser
   745 750 755 760
   Val Ser Thr Thr Thr Ala Glu Ile Lys Asp Leu Ser Glu Asn Lys Leu
   765 770 775
   Pro Val Ile Tyr Met His Val Pro Lys Ser Gly Ala Leu Asn Gln Lys
   780 785 790
   Val Val Phe Tyr Gly Lys Gly Thr Tyr Asp Pro Asp Gly Ser Ile Ala
   795 800 805
   Gly Tyr Gln Trp Asp Phe Gly Asp Gly Ser Asp Phe Ser Ser Glu Gln
   810 815 820
   Asn Pro Ser His Val Tyr Thr Lys Lys Gly Glu Tyr Thr Val Thr Leu
   825 830 835 840
   Arg Val Met Asp Ser Ser Gly Gln Met Ser Glu Lys Thr Met Lys Ile
   845 850 855
   Lys Ile Thr Asp Pro Val Tyr Pro Ile Gly Thr Glu Lys Glu Pro Asn
   860 865 870
   Asn Ser Lys Glu Thr Ala Ser Gly Pro Ile Val Pro Gly Ile Pro Val
   875 880 885
   Ser Gly Thr Ile Glu Asn Thr Ser Asp Gln Asp Tyr Phe Tyr Phe Asp
   890 895 900
   Val Ile Thr Pro Gly Glu Val Lys Ile Asp Ile Asn Lys Leu Gly Tyr
   905 910 915 920
   Gly Gly Ala Thr Trp Val Val Tyr Asp Glu Asn Asn Asn Ala Val Ser
   925 930 935
   Tyr Ala Thr Asp Asp Gly Gln Asn Leu Ser Gly Lys Phe Lys Ala Asp
   940 945 950
   Lys Pro Gly Arg Tyr Tyr Ile His Leu Tyr Met Phe Asn Gly Ser Tyr
   955 960 965
   Met Pro Tyr Arg Ile Asn Ile Glu Gly Ser Val Ser Arg
   970 975 980
<210> 7
   <211> 1034
   <212> PRT
   <213> Clostridium histolyticum
<220>
   <221> SIGNAL
   <222> (-40)..(-1)
<220>
   <221> mat_peptide
   <222> (1)..(994)
<400> 7
   Met Lys Arg Lys Cys Leu Ser Lys Arg Leu Met Leu Ala Ile Thr Met
   -40 -35 -30 -25
   Ala Thr Ile Phe Thr Val Asn Ser Thr Leu Pro Ile Tyr Ala Ala Val
   -20 -15 -10
   Asp Lys Asn Asn Ala Thr Ala Ala Val Gln Asn Glu Ser Lys Arg Tyr
   -5 -1 1 5
   Thr Val Ser Tyr Leu Lys Thr Leu Asn Tyr Tyr Asp Leu Val Asp Leu
   10 15 20
   Leu Val Lys Thr Glu Ile Glu Asn Leu Pro Asp Leu Phe Gln Tyr Ser
   25 30 35 40
   Ser Asp Ala Lys Glu Phe Tyr Gly Asn Lys Thr Arg Met Ser Phe Ile
   45 50 55
   Met Asp Glu Ile Gly Arg Arg Ala Pro Gln Tyr Thr Glu Ile Asp His
   60 65 70
   Lys Gly Ile Pro Thr Leu Val Glu Val Val Arg Ala Gly Phe Tyr Leu
   75 80 85
   Gly Phe His Asn Lys Glu Leu Asn Glu Ile Asn Lys Arg Ser Phe Lys
   90 95 100
   Glu Arg Val Ile Pro Ser Ile Leu Ala Ile Gln Lys Asn Pro Asn Phe
   105 110 115 120
   Lys Leu Gly Thr Glu Val Gln Asp Lys Ile Val Ser Ala Thr Gly Leu
   125 130 135
   Leu Ala Gly Asn Glu Thr Ala Pro Pro Glu Val Val Asn Asn Phe Thr
   140 145 150
   Pro Ile Leu Gln Asp Cys Ile Lys Asn Ile Asp Arg Tyr Ala Leu Asp
   155 160 165
   Asp Leu Lys Ser Lys Ala Leu Phe Asn Val Leu Ala Ala Pro Thr Tyr
   170 175 180
   Asp Ile Thr Glu Tyr Leu Arg Ala Thr Lys Glu Lys Pro Glu Asn Thr
   185 190 195 200
   Pro Trp Tyr Gly Lys Ile Asp Gly Phe Ile Asn Glu Leu Lys Lys Leu
   205 210 215
   Ala Leu Tyr Gly Lys Ile Asn Asp Asn Asn Ser Trp Ile Ile Asp Asn
   220 225 230
   Gly Ile Tyr His Ile Ala Pro Leu Gly Lys Leu His Ser Asn Asn Lys
   235 240 245
   Ile Gly Ile Glu Thr Leu Thr Glu Val Met Lys Val Tyr Pro Tyr Leu
   250 255 260
   Ser Met Gln His Leu Gln Ser Ala Asp Gln Ile Lys Arg His Tyr Asp
   265 270 275 280
   Ser Lys Asp Ala Glu Gly Asn Lys Ile Pro Leu Asp Lys Phe Lys Lys
   285 290 295
   Glu Gly Lys Glu Lys Tyr Cys Pro Lys Thr Tyr Thr Phe Asp Asp Gly
   300 305 310
   Lys Val Ile Ile Lys Ala Gly Ala Arg Val Glu Glu Glu Lys Val Lys
   315 320 325
   Arg Leu Tyr Trp Ala Ser Lys Glu Val Asn Ser Gln Phe Phe Arg Val
   330 335 340
   Tyr Gly Ile Asp Lys Pro Leu Glu Glu Gly Asn Pro Asp Asp Ile Leu
   345 350 355 360
   Thr Met Val Ile Tyr Asn Ser Pro Glu Glu Tyr Lys Leu Asn Ser Val
   365 370 375
   Leu Tyr Gly Tyr Asp Thr Asn Asn Gly Gly Met Tyr Ile Glu Pro Glu
   380 385 390
   Gly Thr Phe Phe Thr Tyr Glu Arg Glu Ala Gln Glu Ser Thr Tyr Thr
   395 400 405
   Leu Glu Glu Leu Phe Arg His Glu Tyr Thr His Tyr Leu Gln Gly Arg
   410 415 420
   Tyr Ala Val Pro Gly Gln Trp Gly Arg Thr Lys Leu Tyr Asp Asn Asp
   425 430 435 440
   Arg Leu Thr Trp Tyr Glu Glu Gly Gly Ala Glu Leu Phe Ala Gly Ser
   445 450 455
   Thr Arg Thr Ser Gly Ile Leu Pro Arg Lys Ser Ile Val Ser Asn Ile
   460 465 470
   His Asn Thr Thr Arg Asn Asn Arg Tyr Lys Leu Ser Asp Thr Val His
   475 480 485
   Ser Lys Tyr Gly Ala Ser Phe Glu Phe Tyr Asn Tyr Ala Cys Met Phe
   490 495 500
   Met Asp Tyr Met Tyr Asn Lys Asp Met Gly Ile Leu Asn Lys Leu Asn
   505 510 515 520
   Asp Leu Ala Lys Asn Asn Asp Val Asp Gly Tyr Asp Asn Tyr Ile Arg
   525 530 535
   Asp Leu Ser Ser Asn Tyr Ala Leu Asn Asp Lys Tyr Gln Asp His Met
   540 545 550
   Gln Glu Arg Ile Asp Asn Tyr Glu Asn Leu Thr Val Pro Phe Val Ala
   555 560 565
   Asp Asp Tyr Leu Val Arg His Ala Tyr Lys Asn Pro Asn Glu Ile Tyr
   570 575 580
   Ser Glu Ile Ser Glu Val Ala Lys Leu Lys Asp Ala Lys Ser Glu Val
   585 590 595 600
   Lys Lys Ser Gln Tyr Phe Ser Thr Phe Thr Leu Arg Gly Ser Tyr Thr
   605 610 615
   Gly Gly Ala Ser Lys Gly Lys Leu Glu Asp Gln Lys Ala Met Asn Lys
   620 625 630
   Phe Ile Asp Asp Ser Leu Lys Lys Leu Asp Thr Tyr Ser Trp Ser Gly
   635 640 645
   Tyr Lys Thr Leu Thr Ala Tyr Phe Thr Asn Tyr Lys Val Asp Ser Ser
   650 655 660
   Asn Arg Val Thr Tyr Asp Val Val Phe His Gly Tyr Leu Pro Asn Glu
   665 670 675 680
   Gly Asp Ser Lys Asn Ser Leu Pro Tyr Gly Lys Ile Asn Gly Thr Tyr
   685 690 695
   Lys Gly Thr Glu Lys Glu Lys Ile Lys Phe Ser Ser Glu Gly Ser Phe
   700 705 710
   Asp Pro Asp Gly Lys Ile Val Ser Tyr Glu Trp Asp Phe Gly Asp Gly
   715 720 725
   Asn Lys Ser Asn Glu Glu Asn Pro Glu His Ser Tyr Asp Lys Val Gly
   730 735 740
   Thr Tyr Thr Val Lys Leu Lys Val Thr Asp Asp Lys Gly Glu Ser Ser
   745 750 755 760
   Val Ser Thr Thr Thr Ala Glu Ile Lys Asp Leu Ser Glu Asn Lys Leu
   765 770 775
   Pro Val Ile Tyr Met His Val Pro Lys Ser Gly Ala Leu Asn Gln Lys
   780 785 790
   Val Val Phe Tyr Gly Lys Gly Thr Tyr Asp Pro Asp Gly Ser Ile Ala
   795 800 805
   Gly Tyr Gln Trp Asp Phe Gly Asp Gly Ser Asp Phe Ser Ser Glu Gln
   810 815 820
   Asn Pro Ser His Val Tyr Thr Lys Lys Gly Glu Tyr Thr Val Thr Leu
   825 830 835 840
   Arg Val Met Asp Ser Ser Gly Gln Met Ser Glu Lys Thr Met Lys Ile
   845 850 855
   Lys Ile Thr Asp Pro Val Tyr Pro Ile Gly Thr Glu Lys Glu Pro Asn
   860 865 870
   Asn Ser Lys Glu Thr Ala Ser Gly Pro Ile Val Pro Gly Ile Pro Val
   875 880 885
   Ser Gly Thr Ile Glu Asn Thr Ser Asp Gln Asp Tyr Phe Tyr Phe Asp
   890 895 900
   Val Ile Thr Pro Gly Glu Val Lys Ile Asp Ile Asn Lys Leu Gly Tyr
   905 910 915 920
   Gly Gly Ala Thr Trp Val Val Tyr Asp Glu Asn Asn Asn Ala Val Ser
   925 930 935
   Tyr Ala Thr Asp Asp Gly Gln Asn Leu Ser Gly Lys Phe Lys Ala Asp
   940 945 950
   Lys Pro Gly Arg Tyr Tyr Ile His Leu Tyr Met Phe Asn Gly Ser Tyr
   955 960 965
   Met Pro Tyr Arg Ile Asn Ile Glu Gly Ser Val Ser Arg Lys Leu Ala
   970 975 980
   Ala Ala Leu Glu His His His His His His
   985 990
<210> 8
   <211> 3105
   <212> DNA
   <213> Clostridium histolyticum
<220>
   <221> CDS
   <222> (1)..(3105)
<400> 8
   atg aaa agg aaa tgt tta tct aaa agg ctt atg tta gct ata aca atg 48
   Met Lys Arg Lys Cys Leu Ser Lys Arg Leu Met Leu Ala Ile Thr Met
   1 5 10 15
   gct aca ata ttt aca gtg aac agt aca tta cca att tat gca gct gta 96
   Ala Thr Ile Phe Thr Val Asn Ser Thr Leu Pro Ile Tyr Ala Ala Val
   20 25 30
   gat aaa aat aat gca aca gca gct gta caa aat gaa agt aag agg tat 144
   Asp Lys Asn Asn Ala Thr Ala Ala Val Gln Asn Glu Ser Lys Arg Tyr
   35 40 45
   aca gta tca tat tta aag act tta aat tat tat gac tta gta gat ttg 192
   Thr Val Ser Tyr Leu Lys Thr Leu Asn Tyr Tyr Asp Leu Val Asp Leu
   50 55 60
   ctt gtt aag act gaa att gag aat tta cca gac ctt ttt cag tat agt 240
   Leu Val Lys Thr Glu Ile Glu Asn Leu Pro Asp Leu Phe Gln Tyr Ser
   65 70 75 80
   tca gat gca aaa gag ttc tat gga aat aaa act cgt atg agc ttt atc 288
   Ser Asp Ala Lys Glu Phe Tyr Gly Asn Lys Thr Arg Met Ser Phe Ile
   85 90 95
   atg gat gaa att ggt aga agg gca cct cag tat aca gag ata gat cat 336
   Met Asp Glu Ile Gly Arg Arg Ala Pro Gln Tyr Thr Glu Ile Asp His
   100 105 110
   aaa ggt att cct act tta gta gaa gtt gta aga gct gga ttt tac tta 384
   Lys Gly Ile Pro Thr Leu Val Glu Val Val Arg Ala Gly Phe Tyr Leu
   115 120 125
   gga ttc cat aac aag gaa ttg aat gaa ata aac aag agg tct ttt aaa 432
   Gly Phe His Asn Lys Glu Leu Asn Glu Ile Asn Lys Arg Ser Phe Lys
   130 135 140
   gaa agg gta ata cct tct ata tta gca att caa aaa aat cct aat ttt 480
   Glu Arg Val Ile Pro Ser Ile Leu Ala Ile Gln Lys Asn Pro Asn Phe
   145 150 155 160
   aaa cta ggt act gaa gtt caa gat aaa ata gta tct gca aca gga ctt 528
   Lys Leu Gly Thr Glu Val Gln Asp Lys Ile Val Ser Ala Thr Gly Leu
   165 170 175
   tta gct ggt aat gaa aca gcg cct cca gaa gtt gta aat aat ttt aca 576
   Leu Ala Gly Asn Glu Thr Ala Pro Pro Glu Val Val Asn Asn Phe Thr
   180 185 190
   cca ata ctt caa gac tgt ata aag aat ata gac aga tac gct ctt gat 624
   Pro Ile Leu Gln Asp Cys Ile Lys Asn Ile Asp Arg Tyr Ala Leu Asp
   195 200 205
   gat tta aag tca aaa gca tta ttt aat gtt tta gct gca cct acc tat 672
   Asp Leu Lys Ser Lys Ala Leu Phe Asn Val Leu Ala Ala Pro Thr Tyr
   210 215 220
   gat ata act gag tat tta aga gct act aaa gaa aaa cca gaa aac act 720
   Asp Ile Thr Glu Tyr Leu Arg Ala Thr Lys Glu Lys Pro Glu Asn Thr
   225 230 235 240
   cct tgg tat ggt aaa ata gat ggg ttt ata aat gaa ctt aaa aag tta 768
   Pro Trp Tyr Gly Lys Ile Asp Gly Phe Ile Asn Glu Leu Lys Lys Leu
   245 250 255
   gct ctt tat gga aaa ata aat gat aat aac tct tgg ata ata gat aac 816
   Ala Leu Tyr Gly Lys Ile Asn Asp Asn Asn Ser Trp Ile Ile Asp Asn
   260 265 270
   ggt ata tat cat ata gca cct tta ggg aag tta cat agc aat aat aaa 864
   Gly Ile Tyr His Ile Ala Pro Leu Gly Lys Leu His Ser Asn Asn Lys
   275 280 285
   ata gga ata gaa act tta aca gag gtt atg aaa gtt tat cct tat tta 912
   Ile Gly Ile Glu Thr Leu Thr Glu Val Met Lys Val Tyr Pro Tyr Leu
   290 295 300
   agt atg caa cat tta caa tca gca gat caa att aag cgt cat tat gat 960
   Ser Met Gln His Leu Gln Ser Ala Asp Gln Ile Lys Arg His Tyr Asp
   305 310 315 320
   tca aaa gat gct gaa gga aac aaa ata cct tta gat aag ttt aaa aag 1008
   Ser Lys Asp Ala Glu Gly Asn Lys Ile Pro Leu Asp Lys Phe Lys Lys
   325 330 335
   gaa gga aaa gaa aaa tac tgt cca aaa act tat aca ttt gat gat gga 1056
   Glu Gly Lys Glu Lys Tyr Cys Pro Lys Thr Tyr Thr Phe Asp Asp Gly
   340 345 350
   aaa gta ata ata aaa gct ggt gct aga gta gaa gaa gaa aaa gtt aaa 1104
   Lys Val Ile Ile Lys Ala Gly Ala Arg Val Glu Glu Glu Lys Val Lys
   355 360 365
   aga cta tac tgg gca tca aag gaa gtt aac tct caa ttc ttt aga gta 1152
   Arg Leu Tyr Trp Ala Ser Lys Glu Val Asn Ser Gln Phe Phe Arg Val
   370 375 380
   tac gga ata gac aaa cca tta gaa gaa ggt aat cca gat gat ata tta 1200
   Tyr Gly Ile Asp Lys Pro Leu Glu Glu Gly Asn Pro Asp Asp Ile Leu
   385 390 395 400
   aca atg gtt atc tac aac agt ccc gaa gaa tat aaa ctc aat agt gtt 1248
   Thr Met Val Ile Tyr Asn Ser Pro Glu Glu Tyr Lys Leu Asn Ser Val
   405 410 415
   cta tac gga tat gat act aat aat ggt ggt atg tat ata gag cca gaa 1296
   Leu Tyr Gly Tyr Asp Thr Asn Asn Gly Gly Met Tyr Ile Glu Pro Glu
   420 425 430
   gga act ttc ttc acc tat gaa aga gaa gct caa gaa agc aca tac aca 1344
   Gly Thr Phe Phe Thr Tyr Glu Arg Glu Ala Gln Glu Ser Thr Tyr Thr
   435 440 445
   tta gaa gaa tta ttt aga cat gaa tat aca cat tat ttg caa gga aga 1392
   Leu Glu Glu Leu Phe Arg His Glu Tyr Thr His Tyr Leu Gln Gly Arg
   450 455 460
   tat gca gtt cca gga caa tgg gga aga aca aaa ctt tat gac aat gat 1440
   Tyr Ala Val Pro Gly Gln Trp Gly Arg Thr Lys Leu Tyr Asp Asn Asp
   465 470 475 480
   aga tta act tgg tat gaa gaa ggt gga gca gaa tta ttt gca ggt tct 1488
   Arg Leu Thr Trp Tyr Glu Glu Gly Gly Ala Glu Leu Phe Ala Gly Ser
   485 490 495
   act aga act tct gga ata tta cca aga aag agt ata gta tca aat att 1536
   Thr Arg Thr Ser Gly Ile Leu Pro Arg Lys Ser Ile Val Ser Asn Ile
   500 505 510
   cat aat aca aca aga aat aat aga tat aag ctt tca gac act gta cat 1584
   His Asn Thr Thr Arg Asn Asn Arg Tyr Lys Leu Ser Asp Thr Val His
   515 520 525
   tct aaa tat ggt gct agt ttt gaa ttc tat aat tat gca tgt atg ttt 1632
   Ser Lys Tyr Gly Ala Ser Phe Glu Phe Tyr Asn Tyr Ala Cys Met Phe
   530 535 540
   atg gat tat atg tat aat aaa gat atg ggt ata tta aat aaa cta aat 1680
   Met Asp Tyr Met Tyr Asn Lys Asp Met Gly Ile Leu Asn Lys Leu Asn
   545 550 555 560
   gat ctt gca aaa aat aat gat gtt gat gga tat gat aat tat att aga 1728
   Asp Leu Ala Lys Asn Asn Asp Val Asp Gly Tyr Asp Asn Tyr Ile Arg
   565 570 575
   gat tta agt tct aat tat gct tta aat gat aaa tat caa gat cat atg 1776
   Asp Leu Ser Ser Asn Tyr Ala Leu Asn Asp Lys Tyr Gln Asp His Met
   580 585 590
   cag gag cgc ata gat aat tat gaa aat tta aca gtg cct ttt gta gct 1824
   Gln Glu Arg Ile Asp Asn Tyr Glu Asn Leu Thr Val Pro Phe Val Ala
   595 600 605
   gat gat tat tta gta agg cat gct tat aag aac cct aat gaa att tat 1872
   Asp Asp Tyr Leu Val Arg His Ala Tyr Lys Asn Pro Asn Glu Ile Tyr
   610 615 620
   tct gaa ata tct gaa gta gca aaa tta aag gat gct aag agt gaa gtt 1920
   Ser Glu Ile Ser Glu Val Ala Lys Leu Lys Asp Ala Lys Ser Glu Val
   625 630 635 640
   aag aaa tca caa tat ttt agt acc ttt act ttg aga ggt agt tac aca 1968
   Lys Lys Ser Gln Tyr Phe Ser Thr Phe Thr Leu Arg Gly Ser Tyr Thr
   645 650 655
   ggt gga gca tct aag ggg aaa tta gaa gat caa aaa gca atg aat aag 2016
   Gly Gly Ala Ser Lys Gly Lys Leu Glu Asp Gln Lys Ala Met Asn Lys
   660 665 670
   ttt ata gat gat tca ctt aag aaa tta gat acg tat tct tgg agt ggg 2064
   Phe Ile Asp Asp Ser Leu Lys Lys Leu Asp Thr Tyr Ser Trp Ser Gly
   675 680 685
   tat aaa act tta act gct tat ttc act aat tat aaa gtt gac tct tca 2112
   Tyr Lys Thr Leu Thr Ala Tyr Phe Thr Asn Tyr Lys Val Asp Ser Ser
   690 695 700
   aat aga gtt act tat gat gta gta ttc cac gga tat tta cca aac gaa 2160
   Asn Arg Val Thr Tyr Asp Val Val Phe His Gly Tyr Leu Pro Asn Glu
   705 710 715 720
   ggt gat tcc aaa aat tca tta cct tat ggc aag atc aat gga act tac 2208
   Gly Asp Ser Lys Asn Ser Leu Pro Tyr Gly Lys Ile Asn Gly Thr Tyr
   725 730 735
   aag gga aca gag aaa gaa aaa atc aaa ttc tct agt gaa ggc tct ttc 2256
   Lys Gly Thr Glu Lys Glu Lys Ile Lys Phe Ser Ser Glu Gly Ser Phe
   740 745 750
   gat cca gat ggt aaa ata gtt tct tat gaa tgg gat ttc gga gat ggt 2304
   Asp Pro Asp Gly Lys Ile Val Ser Tyr Glu Trp Asp Phe Gly Asp Gly
   755 760 765
   aat aag agt aat gag gaa aat cca gag cat tca tat gac aag gta gga 2352
   Asn Lys Ser Asn Glu Glu Asn Pro Glu His Ser Tyr Asp Lys Val Gly
   770 775 780
   act tat aca gtg aaa tta aaa gtt act gat gac aag gga gaa tct tca 2400
   Thr Tyr Thr Val Lys Leu Lys Val Thr Asp Asp Lys Gly Glu Ser Ser
   785 790 795 800
   gta tct act act act gca gaa ata aag gat ctt tca gaa aat aaa ctt 2448
   Val Ser Thr Thr Thr Ala Glu Ile Lys Asp Leu Ser Glu Asn Lys Leu
   805 810 815
   cca gtt ata tat atg cat gta cct aaa tcc gga gcc tta aat caa aaa 2496
   Pro Val Ile Tyr Met His Val Pro Lys Ser Gly Ala Leu Asn Gln Lys
   820 825 830
   gtt gtt ttc tat gga aaa gga aca tat gac cca gat gga tct atc gca 2544
   Val Val Phe Tyr Gly Lys Gly Thr Tyr Asp Pro Asp Gly Ser Ile Ala
   835 840 845
   gga tat caa tgg gac ttt ggt gat gga agt gat ttt agc agt gaa caa 2592
   Gly Tyr Gln Trp Asp Phe Gly Asp Gly Ser Asp Phe Ser Ser Glu Gln
   850 855 860
   aac cca agc cat gta tat act aaa aaa ggt gaa tat act gta aca tta 2640
   Asn Pro Ser His Val Tyr Thr Lys Lys Gly Glu Tyr Thr Val Thr Leu
   865 870 875 880
   aga gta atg gat agt agt gga caa atg agt gaa aaa act atg aag att 2688
   Arg Val Met Asp Ser Ser Gly Gln Met Ser Glu Lys Thr Met Lys Ile
   885 890 895
   aag att aca gat ccg gta tat cca ata ggc act gaa aaa gaa cca aat 2736
   Lys Ile Thr Asp Pro Val Tyr Pro Ile Gly Thr Glu Lys Glu Pro Asn
   900 905 910
   aac agt aaa gaa act gca agt ggt cca ata gta cca ggt ata cct gtt 2784
   Asn Ser Lys Glu Thr Ala Ser Gly Pro Ile Val Pro Gly Ile Pro Val
   915 920 925
   agt gga acc ata gaa aat aca agt gat caa gat tat ttc tat ttt gat 2832
   Ser Gly Thr Ile Glu Asn Thr Ser Asp Gln Asp Tyr Phe Tyr Phe Asp
   930 935 940
   gtt ata aca cca gga gaa gta aaa ata gat ata aat aaa tta ggg tac 2880
   Val Ile Thr Pro Gly Glu Val Lys Ile Asp Ile Asn Lys Leu Gly Tyr
   945 950 955 960
   gga gga gct act tgg gta gta tat gat gaa aat aat aat gca gta tct 2928
   Gly Gly Ala Thr Trp Val Val Tyr Asp Glu Asn Asn Asn Ala Val Ser
   965 970 975
   tat gcc act gat gat ggg caa aat tta agt gga aag ttt aag gca gat 2976
   Tyr Ala Thr Asp Asp Gly Gln Asn Leu Ser Gly Lys Phe Lys Ala Asp
   980 985 990
   aaa cca ggt aga tat tac atc cat ctt tac atg ttt aat ggt agt tat 3024
   Lys Pro Gly Arg Tyr Tyr Ile His Leu Tyr Met Phe Asn Gly Ser Tyr
   995 1000 1005
   atg cca tat aga att aat ata gaa ggt tca gta tct aga aag ctt 3069
   Met Pro Tyr Arg Ile Asn Ile Glu Gly Ser Val Ser Arg Lys Leu
   1010 1015 1020
   gcg gcc gca ctc gag cac cac cac cac cac cac tga 3105
   Ala Ala Ala Leu Glu His His His His His His
   1025 1030
<210> 9
   <211> 25
   <212> DNA
   <213> Artificial
<220>
   <223> A primer for PCR (colG-F)
<400> 9
   atgaaaaaaa atattttaaa gattc 25
<210> 10
   <211> 27
   <212> DNA
   <213> Artificial
<220>
   <223> A primer for PCR (colG-R)
<400> 10
   ccggatccta tctagatacc cttaact 27
<210> 11
   <211> 28
   <212> DNA
   <213> Artificial
<220>
   <223> A primer for PCR (His-F)
<400> 11
   gctctagaaa gcttgcggcc gcactcga 28
<210> 12
   <211> 24
   <212> DNA
   <213> Artificial
<220>
   <223> A primer for PCR (His-R)
<400> 12
   cgggatccgg atatagttcc tcct 24
<210> 13
   <211> 28
   <212> DNA
   <213> Artificial
<220>
   <223> A primer for PCR (lac-F)
<400> 13
   ccggcaagct tgcccaatac gcaaaccg 28
<210> 14
   <211> 18
   <212> DNA
   <213> Artificial
<220>
   <223> A primer for PCR (lac-R)
<400> 14
   agctgtttcc tgtgtgaa 18
<210> 15
   <211> 20
   <212> DNA
   <213> Artificial
<220>
   <223> A primer for PCR (colH-F)
<400> 15
   atgaaaagga aatgtttatc 20
<210> 16
   <211> 27
   <212> DNA
   <213> Artificial
<220>
   <223> A primer for PCR (colH-R)
<400> 16
   ccggatccta tctagatact gaacctt 27

## Description

### [Technical Field]

The present invention relates to a collagenase used for isolating cells (cell mass) from an organ, such as cells of pancreatic islets from a pancreas.

### [Background Art]

As a curative therapy for diabetes, pancreatic islet transplantation has been known, in which pancreatic islets (insulin-producing cells) isolated from a pancreas treated with a protease are transplanted into the portal vein of a diabetic patient via intraverous drip. This transplantation method does not require laparotomy for the patient during the transplantation, and therefore recently has attracted attention as a curative therapy, which is safe and simple, for diabetes.

Pancreatic islets are isolated from a pancreas by treating the pancreas with a collagenase and a neutral metalloprotease. As the collagenase used for this purpose, a collagenase derived from Clostridium histolyticum is particularly effective (Non-Patent Literatures 1, 2).

The collagenase derived from Clostridium histolyticum has been known to include two types of enzyme having different substrate specificity; collagenase G and collagenase H (Non-Patent Literatures 1, 2). Genes encoding collagenase G and collagenase H have been already isolated, and both collagenases have been found to be multi-domain enzymes including a catalytic domain on the amino terminal side and a collagen-binding domain (hereinafter, referred to as "CBD") on the carboxyl terminal side (Non-Patent Literatures 1, 2).

However, there has been a problem that, when a collagenase is produced by Clostridium histolyticum, part of the expressed collagenase is degraded (Non-Patent Literature 2), and treating of a pancreas with a collagenase mixed with the degraded collagenase decreases the quality of isolated pancreatic islets. Thus, for isolation of pancreatic islets, it is desirable to treat a pancreas with a collagenase obtained by removing such a degraded collagenase as much as possible therefrom.

However, a non-degraded collagenase and a degraded collagenase are similar in physicochemical properties, and therefore it has been difficult to separate these collagenases by methods such as ion-exchange chromatography or hydrophobic chromatography.

In such backgrounds, a method has been desired for preparing a collagenase by selectively collecting a non-degraded collagenase from collagenases derived from Clostridium histolyticum.

It should be noted that degradation of a collagenase derived from Clostridium histolyticum has not been reported yet with the collagenase being expressed as a recombinant protein in a host such as E. coli.

### [Citation List]

### [Non Patent Literatures]

[NPL 1] Yoshihara, K. et. al. Journal of Bacteriology. (1994), 176, 6489-6496
[NPL 2] Matsushita, O. et. al. Journal of Bacteriology. (1999), 181, 923-933

### [Summary of Invention]

BIOMOLECULAR NMR ASSIGNMENTS, vol.2, no. 2, p. 127-129 discloses a fusion protein comprising a collagenase from C. histolyticum and a GST tag. The C-terminal collagen-binding domain of the collagenase is fused to the C-terminal of the GST tag. APL MICROBIOL BIOTECHNOL (2008) 19:627-635 describes a fusion collagenase in which a six Histidin containing affinity tag is linked to the carboxyl terminals of the collagenase expressed in Clostridium perfringens.

### [Technical Problem]

The present invention aims at selectively collecting a non-degraded collagenase by removing a degraded collagenase from collagenases derived from Clostridium histolyticum.

### [Solution to Problem]

With respect to collagenases derived from Clostridium histolyticum, fusion collagenases in which affinity tags linked to the carboxyl terminals of the collagenases were expressed as recombinant proteins by the present inventors. When a collagenase derived from Clostridium histolyticum is expressed as a recombinant protein in a host such as E. coli, it has not been well-predicted whether the recombinant protein is degraded by the action of a protease in a host or not, and how the recombinant protein is degraded supposing that it is degraded. The present inventors, however, purified the fusion protein expressed in a host with an affinity column, and accidentally found that a degraded collagenase was removed and that a single collagenase having a collagenase activity was selectively collected. This is based on the following assumptions that: a collagenase derived from Clostridium histolyticum expressed in a host was degraded at the CBD in the neighborhood of the fused affinity tag, and therefore degradation of the CBD allowed the affinity tag to be separated from the collagenase; and in the purification stage by affinity chromatography, the degraded collagenase was not adsorbed to the affinity column but only a non-degraded collagenase was adsorbed to the affinity column.

In other words, the present inventors found out that if an affinity tag is linked to a collagenase in a specific configuration, a single collagenase having a collagenase activity can be selectively collected in the affinity purification process without the collagenase degraded in a host being adsorbed. This discovery has led the inventors to the completion of the present invention.

The present invention relates to a method for producing a fusion collagenase, wherein:
a culture obtained by culturing E. coli transformed by any one of a DNA encoding the fusion collagenase and an expression vector comprising the DNA is purified by affinity chromatography corresponding to an affinity tag to thereby selectively collect the fusion collagenase having a collagen-binding domain, wherein the fusion collagenase has the following features (i) to (ii) :
   (i)the affinity tag is directly or indirectly linked to a carboxyl terminal of the collagenase;
   (ii) the collagenase is selected from the following (a) to (h) :
      (a) a collagenase comprising an amino acid sequence from positions -110 to 1008 of any one of SEQ ID NOS: 1 and 2;
      (b) a collagenase comprising an amino acid sequence from positions -110 to 1008 of any one of SEQ ID NOS: 1 and 2 in which 1 to 30 amino acids are deleted, substituted, inserted, or added;
      (c) a collagenase comprising an amino acid sequence having a homology of 80% or more with the amino acid sequence from positions -110 to 1008 of any one of SEQ ID NOS: 1 and 2 ;
      (d) a collagenase in which an amino acid sequence from positions -110 to -1 of any one of SEQ ID NOS: 1 and 2 is removed from any one of the collagenases described in (a) to (c) ;
      (e) a collagenase comprising an amino acid sequence from positions -40 to 981 of any one of SEQ ID NOS: 5 and 6;
      (f) a collagenase comprising an amino acid sequence from positions -40 to 981 of any one of SEQ ID NOS: 5 and 6 in which 1 to 30 amino acids are deleted, substituted, inserted, or added;
      (g) a collagenase comprising an amino acid sequence having a homology of 80% or more with the amino acid sequence from positions -40 to 981 of any one of SEQ ID NOS: 5 and 6;
      (h) a collagenase in which an amino acid sequence from positions -40 to -1 of any one of SEQ ID NOS: 5 and 6 is removed from any one of the collagenases described in (e) to (g).

The fusion collagenase according to the invention, wherein the affinity tag is directly or indirectly linked to a carboxyl terminal of the collagenase.

The fusion collagenase according to the invention, wherein the affinity tag is two or more consecutive histidine residues.

The fusion collagenase according to the invention, wherein the collagenase is derived from Clostridium histolyticum.

The fusion collagenase according to the invention may comprise an amino acid sequence from positions -110 to 1021 of SEQ ID NO: 3. In particular, from the aforementioned fusion collagenase an amino acid sequence from positions -110 to -1 of SEQ ID NO: 3 is removed.

The fusion collagenase according to the invention, comprises an amino acid sequence from positions -40 to 994 of SEQ ID NO: 7. In particular, from the aforementioned fusion collagenase an amino acid sequence from positions -40 to -1 of SEQ ID NO: 7 is removed.

Described herein is a DNA encoding the fusion collagenase according to the invention.

Described herein is a DNA comprising a base sequence from positions 1 to 3396 of SEQ ID NO: 4.

Described herein is a DNA comprising a base sequence from positions 1 to 3105 of SEQ ID NO: 8.

Described herein is an expression vector comprising the aforementioned DNA.

Further described herein is a host cell transformed by any one of the aforementioned DNA and the expression vector. The host cell is E. coli.

Described herein is a fusion collagenase produced by the method according to the invention.

### [Advantageous Effect of Invention]

The present invention provides a fusion collagenase in which an affinity tag is linked to a collagenase derived from Clostridium histolyticum, wherein the collagenase and the affinity tag are linked to each other in such a manner that a fragment having a collagenase activity and the affinity tag are separated from each other when the fusion collagenase expressed in a host is degraded by an action of the host.

The present invention also provides a DNA required to produce the fusion collagenase as a recombinant protein efficiently, and a host cell expressing the fusion collagenase as a recombinant protein. In addition, the present invention provides a method capable of selectively collecting a single fusion collagenase having a CBD by removing a collagenase in which part or all of a CBD is degraded from a culture solution obtained by culturing a host cell expressing the fusion collagenase. The present invention leads to efficient production of a fusion collagenase having a CBD.

### [Brief Description of Drawings]

[Fig. 1] Fig. 1 is a drawing showing a structure of a plasmid pColG. In the drawing, the meanings of the symbols are as follows (the oblique types in the drawing are underlined). colG: a collagenase G gene, PlacZ: a lacZ promoter, and Amp^{r}: an ampicillin-resistance gene.
[Fig. 2] Fig. 2 is a drawing showing a structure of a plasmid pColG-His. In the drawing, the meanings of the symbols are as follows (the oblique types in the drawing are underlined). colG: a collagenase G gene, PlacZ: a lacZ promoter, Amp^{r}: an ampicillin-resistance gene, MCS: a multi cloning site, and 6XHis: a histidine tag.
[Fig. 3] Fig. 3 is a picture of electrophoresis showing the result of activity staining performed on an extract of E. coli having fusion collagenase G expressed.
[Fig. 4] Fig. 4 is a picture of electrophoresis showing the result of activity staining performed on a solution of fusion collagenase G subjected to affinity chromatography.
[Fig. 5] Fig. 5 is a drawing showing a structure of a plasmid pColH. In the drawing, the meanings of the symbols are as follows (the oblique types in the drawing are underlined). colH: a collagenase H gene, PlacZ: a lacZ promoter, and Amp^{r}: an ampicillin-resistance gene.
[Fig. 6] Fig. 6 is a drawing showing a structure of a plasmid pColH-His. In the drawing, the meanings of the symbols are as follows (the oblique types in the drawing are underlined). colH: a collagenase H gene, PlacZ: a lacZ promoter, Amp^{r}: an ampicillin-resistance gene, MCS: a multi cloning site, and 6XHis: a histidine tag.
[Fig. 7] Fig. 7 is a picture of electrophoresis showing the result of activity staining performed on an extract of E. coli having fusion collagenase H expressed.
[Fig. 8] Fig. 8 is a picture of electrophoresis showing the result of activity staining performed on a solution of fusion collagenase H subjected to affinity chromatography.

### [Description of Embodiments]

### (Fusion collagenase)

In the present invention, a fusion collagenase refers to a protein in which an affinity tag is directly or indirectly linked to a collagenase, i.e. a fusion collagenase in which the collagenase and the affinity tag are linked to each other in such a manner that a fragment having a collagenase activity and the affinity tag in the fusion collagenase are separated from each other when the fusion collagenase expressed in a host is degraded by an action of the host. The linking manner of such a collagenase to the affinity tag is preferably linking to a CBD of the collagenase, and most preferably linking to the carboxyl terminal of the CBD (i.e. the carboxyl terminal of the collagenase). In the present invention, the "CBD" means regions of segments 3a and 3b in collagenase G (position 776 to carboxyl terminal of each of SEQ ID NOS: 1 and 2), and a region of a segment 3 in collagenase H (from position 864 to carboxyl terminal of SEQ ID NO: 5) (Non-Patent Literature 2).

As a collagenase in the present invention, any collagenase can be used as long as pancreatic islets can be isolated from a pancreas by being treated in combination with a neutral metalloprotease. Particularly, the use of a collagenase derived from Clostridium histolyticum is desirable. Any collagenase derived from Clostridium histolyticum can be used, but particularly the use of the collagenase G of any one of SEQ ID NOS: 1 and 2, or the collagenase H of any one of SEQ ID NOS: 5 and 6 is desirable. In addition, such a collagenase may include an amino acid sequence having all or part of a signal sequence removed.

Here, the amino acid sequence of the collagenase G may be any of the followings as long as the collagenase activity and the binding to collagen are retained: (i) a collagenase comprising all or part of an amino acid sequence from positions -110 to 1008 of any one of SEQ ID NOS: 1 and 2; (ii) a collagenase comprising an amino acid sequence from positions -110 to 1008 of any one of SEQ ID NOS: 1 and 2 in which one or more amino acids are deleted, substituted, inserted, or added; (iii) a collagenase comprising an amino acid sequence having a homology of 70% or more with the amino acid sequence from positions -110 to 1008 of any one of SEQ ID NOS: 1 and 2; and (iv) a collagenase in which all or part of a signal sequence including an amino acid sequence from positions -110 to -1 of any one of SEQ ID NOS : 1 and 2 is removed from any one of the collagenases described in (i) to (iii).

Similarly, the amino acid sequence of the collagenase H may be any of the followings as long as the collagenase activity and the binding to collagen are retained: (i) a collagenase comprising all or part of an amino acid sequence from positions -40 to 981 of any one of SEQ ID NOS: 5 and 6; (ii) a collagenase comprising an amino acid sequence from positions -40 to 981 of any one of SEQ ID NOS: 5 and 6 in which one or more amino acids are deleted, substituted, inserted, or added; (iii) a collagenase comprising an amino acid sequence having a homology of 70% or more with the amino acid sequence from positions -40 to 981 of any one of SEQ ID NOS: 5 and 6; and (iv) a collagenase in which all or part of a signal sequence including an amino acid sequence from positions -40 to -1 of any one of SEQ ID NOS: 5 and 6 is removed from any one of the collagenases described in (i) to (iii).

Here, the "amino acid sequence in which one or more amino acids are deleted, substituted, inserted, or added" means that the amino acid sequence is modified by well-known methods such as site-directed mutagenesis, or substitution or the like of amino acids as many as the number of amino acids naturally substituted. The number of modification of amino acids is preferably 1 to 50, more preferably 1 to 30, further preferably 1 to 10, still further preferably 1 to 5, and most preferably 1 to 2. An example of the modified amino acid sequence preferably can be an amino acid sequence having one or more (preferably, 1 to several, or 1, 2, 3, or 4) conservative substitutions of amino acids thereof. Here, the "conservative substitution" means that at least one amino acid residue is substituted with another chemically similar amino acid residue. Examples include a case where a certain hydrophobic residue is substituted with another hydrophobic residue, a case where a certain polar residue is substituted with another polar residue having the same charge, or other cases. Functionally similar amino acids which can be subjected to such substitution are known in the art for each amino acid. Specific examples of non-polar (hydrophobic) amino acids include alanine, valine, isoleucine, leucine, proline, tryptophan, phenylalanine, methionine, etc. Specific examples of polar (neutral) amino acids include glycine, serine, threonine, tyrosine, glutamine, asparagine, cysteine, etc. Specific examples of positively-charged (basic) amino acids include arginine, histidine, lysine, etc. Furthermore, specific examples of negatively-charged (acidic) amino acids include aspartic acid, glutamic acid, etc.

Further, the "amino acid sequence having a homology of 70% or more" can be an amino acid sequence having a homology of, preferably 80% or more, more preferably 85% or more, further preferably 90% or more, still further preferably 95% or more, particularly preferably 98% or more, and most preferably 99% or more. The term "homology" with regard to base sequence or amino acid sequence is used as a meaning of the degree of correspondence, between sequences to be compared, in bases or amino acid residues constituting each sequence. Each of the numerical values of "homology" described in the present description may be any numerical value calculated using a homology search program known to those skilled in the art, and can be easily calculated by using the default (initial setting) parameters in FASTA, BLAST, etc., for example.

In the present invention, as the affinity tag to be directly or indirectly fused to the collagenase, any affinity tag can be used as long as it can be selectively bound to a certain type of carrier. For example, a histidine tag including two or more consecutive histidine residues that are selectively bound to a nickel chelate column described in Japanese Patent No. 2686090, a cellulose-binding domain that is selectively bound to an insoluble cellulose, a maltose-binding domain that is selectively bound to a maltose-binding resin, and the like can be used. Particularly, the use of the histidine tag having a smaller molecular weight is desirable.

In the present invention, the affinity tag is directly or indirectly linked to the collagenase. Here, when the collagenase and the affinity tag are indirectly linked to each other, the amino acid sequence intervening therebetween may be any sequence as long as it does not materially inhibit the activity of the fusion collagenase and the binding to collagen.

Examples of the fusion collagenase that meets the above requirements include a fusion collagenase comprising all of an amino acid sequence of SEQ ID NO: 3 in which the histidine tag is linked to the carboxyl terminal of the collagenase G, and a fusion collagenase comprising all of an amino acid sequence of SEQ ID NO: 7 in which the histidine tag is linked to the carboxyl terminal of the collagenase H. Also, the examples include a fusion collagenase comprising part of such amino acid sequence as long as the collagenase activity, the binding to collagen, and the linking to the affinity tag are retained.

### (DNA encoding amino acid sequence of fusion collagenase)

In the present invention, a DNA encoding the fusion collagenase may be a DNA comprising any base sequences as long as the DNA encodes the amino sequence of the fusion collagenase described above.

The DNA encoding the fusion collagenase of the present invention can be obtained by artificial chemical synthesis. Also, the DNA encoding the fusion collagenase can be obtained by constructing a DNA encoding the collagenase and a DNA encoding the affinity tag separately and linking them together. In this case, the collagenase gene can be amplified by PCR using a primer synthesized based on the sequence of the gene and with a template of a DNA, such as genomic DNA, cDNA, and plasmid, including the gene. For example, the collagenase G or the collagenase H derived from Clostridium histolyticum can be amplified by PCR using a primer designed based on the sequence of 5' end and 3' end of the sequence of the collagenase G gene described in Non-Patent Literature 1 or the collagenase H gene described in Non-Patent Literature 2 and with a template of a genomic DNA derived from Clostridium histolyticum. Moreover, the DNA encoding the affinity tag can be amplified by PCR with a template of a genomic DNA, cDNA, plasmid, or the like including the gene encoding the affinity tag. For example, a DNA encoding the histidine tag can be amplified by PCR with a template of pET-24a(+), a commercially available vector.

Examples of the DNA that meets the above requirements include a DNA comprising all or part of the base sequence of SEQ ID NO: 4 encoding the fusion collagenase in which the histidine tag is linked to the carboxyl terminal of the collagenase G; and a DNA comprising all or part of the base sequence of SEQ ID NO: 8 for linking the histidine tag to the carboxyl terminal of the collagenase H.

### (Expression vector, and host cell transformed by the expression vector)

The present invention provides an expression vector comprising the DNA encoding the amino acid sequence of the fusion collagenase described above, being capable of replicating the DNA in a host cell, and comprising a protein encoded by the DNA sequence in an expressible state. The present expression vector can be constructed based on a self-replicating vector, i.e., for example, a plasmid that exists as an independent extrachromosomal element and replicates independently of the replication of the chromosome. In addition, the present expression vector may be any vector that is introduced into a host cell, then incorporated into the genome of the host cell, and replicated together with the chromosome incorporated therewith. As a procedure and a method for constructing the vector according to the present invention, any procedure and any method commonly used in the field of genetic engineering can be used.

The expression vector according to the present invention, to express the fusion collagenase when the expression vector is actually introduced into the host cell, desirably comprises, in addition to the DNA encoding the amino acid sequence of the fusion collagenase described above, a DNA sequence for regulating the expression of the DNA, a genetic marker for selecting the transformed host cell, and the like. The DNA sequence for regulating the expression includes a DNA sequence encoding a promoter, terminator, and signal peptide, and the like. The promoter is not particularly limited as long as it exhibits the transcriptional activity in a host cell, and can be obtained as the DNA sequence for regulating the expression of the gene encoding either a homogeneous or heterogeneous protein with respect to the host cell. In addition, the signal peptide is not particularly limited as long as it contributes to secretion of the protein in the host cell, and can be obtained from the DNA sequence derived from the gene encoding either a homogeneous or heterogeneous protein with respect to the host cell. Moreover, the genetic marker in the present invention may be appropriately selected depending on the method for selecting the transformant, and a gene encoding for drug resistance and a gene complementing the auxotrophy, for example, can be used.

Further, according to the present invention, a host cell transformed by this expression vector is provided. This host-vector system is not particularly limited, and a system using E. coli, actinomycete, yeast, filamentous fungus, animal cell, etc., for example, or the like can be used. Particularly, E. coli is desirably used as a host.

Also, the host cell can be transformed by such expression vectors according to methods commonly used in the art.

### (Culturing of host cell expressing fusion collagenase, and selective collection of fusion collagenase having CBD by affinity chromatography)

In the present invention, the fusion collagenase can be obtained from a culture which is obtained by culturing the host cell expressing the fusion collagenase can be cultured in a suitable medium. The culturing and its condition for the host cell expressing the fusion collagenase may be substantially the same as those for the host cell used.

The fusion collagenase secreted in the host cell expressing the fusion collagenase or in the culture solution can be collected by adopting methods commonly used in the art.

The fusion collagenase collected according to the above method is subjected to affinity chromatography to remove a degraded collagenase, thereby selectively collecting the fusion collagenase having the CBD. Affinity chromatography used in this case needs to be a method which corresponds to the affinity tag linked to the collagenase. For example, when the histidine tag is linked to the collagenase, a nickel chelate column or the like to which the histidine tag is selectively linked is used for purification.

The extent to which a degraded collagenase is removed by affinity chromatography can be evaluated by subjecting the fusion collagenase solution to electrophoresis using gel with gelatin added thereto and then to activity staining.

### (Other aspects)

According to the knowledge of the present inventors that the expression of a recombinant collagenase by E. coli or the like causes part or all of its CBD to be degraded, the degraded collagenase can be eliminated and a non-degraded recombinant collagenase can be specifically affinity-purified using an antibody that binds to the CBD of the recombinant collagenase. In this case, it is advantageous in that there is no need to fuse the affinity tag to the recombinant collagenase. Specifically, the present invention also provides a method for producing a collagenase, characterized in that a culture obtained by culturing a host cell having a recombinant collagenase expressed is purified with an antibody that binds to a CBD of the recombinant collagenase to thereby selectively collect a collagenase having the CBD. In addition, because the CBD and collagen have an affinity for each other, the recombinant collagenase can be specifically affinity-purified using collagen instead of the antibody described above. Specifically, the present invention also provides a method for producing a collagenase, wherein a culture obtained by culturing a host cell having a recombinant collagenase expressed is purified with collagen to thereby selectively collect a collagenase having the CBD.

### [Examples]

The present invention will be more specifically described by way of Examples, but the present invention is not to be limited to Examples below but is still within the gist of the present invention.

### [Example 1] Expression of fusion collagenase (fusion collagenase G) in which histidine tag is linked to carboxyl terminal of collagenase G derived from Clostridium histolyticum

### (1-1) Preparation of collagenase G gene fragment

A collagenase G gene derived from Clostridium histolyticum was amplified from the 5' end to the 3' end thereof by PCR with a template of a genomic DNA derived from Clostridium histolyticum. In this case, the primers were designed so that the 3' end of the amplified collagenase G gene served as an XbaI-recognition sequence and further that a BamHI-recognition sequence was added subsequent to the stop codon of the gene. As a result, mutations were introduced into the amino acid sequence at two positions (amino acids at positions 1007 and 1008 of SEQ ID NO: 1) on the carboxyl terminal side of natural collagenase G described in Non-Patent Literature 1. Thus, the amino acid sequence was modified to the amino acid sequence of SEQ ID NO: 2.

The primers used in this PCR were as follows.
colG-F:
   ATGAAAAAAAATATTTTAAAGATTC (SEQ ID NO: 9)
colG-R:
   CCGGATCCTATCTAGATACCCTTAACT (SEQ ID NO: 10)

The amplified collagenase G gene fragment was digested with BamHI.

### (1-2) Preparation of DNA fragment including region encoding histidine tag

To prepare a DNA encoding the histidine tag including six consecutive histidine residues, the DNA encoding the histidine tag was amplified by PCR with a template of pET-24a(+), a commercially available vector. In the DNA fragment, in addition to the DNA encoding the histidine tag, a multi cloning site originated from the vector and a gene fragment corresponding to a T7 terminator were included. The primers were designed to include the XbaI-recognition sequence at the 5' end of the amplified DNA fragment and the BamHI-recognition sequence at the 3' end thereof.

The primers used in this PCR were as follows.
His-F:
   GCTCTAGAAAGCTTGCGGCCGCACTCGA (SEQ ID NO: 11)
His-R:
   CGGGATCCGGATATAGTTCCTCCT (SEQ ID NO: 12)

The amplified DNA fragment including the region encoding the histidine tag was double-digested with XbaI and BamHI.

### (1-3) Preparation of lacZ promoter fragment

A lacZ promoter was prepared as a promoter for expressing the fusion collagenase. The DNA fragment was amplified by PCR with a template of pUC19. In this case, the primers were designed to include a HindIII-recognition sequence at the 5' end of the amplified DNA fragment.

The primers used in this PCR were as follows.
lac-F:
   CCGGCAAGCTTGCCCAATACGCAAACCG (SEQ ID NO: 13)
lac-R:
   AGCTGTTTCCTGTGTGAA (SEQ ID NO: 14)

The amplified lacZ promoter fragment was digested with HindIII.

### (1-4) Construction of expression vector for fusion collagenase G to which histidine tag is linked

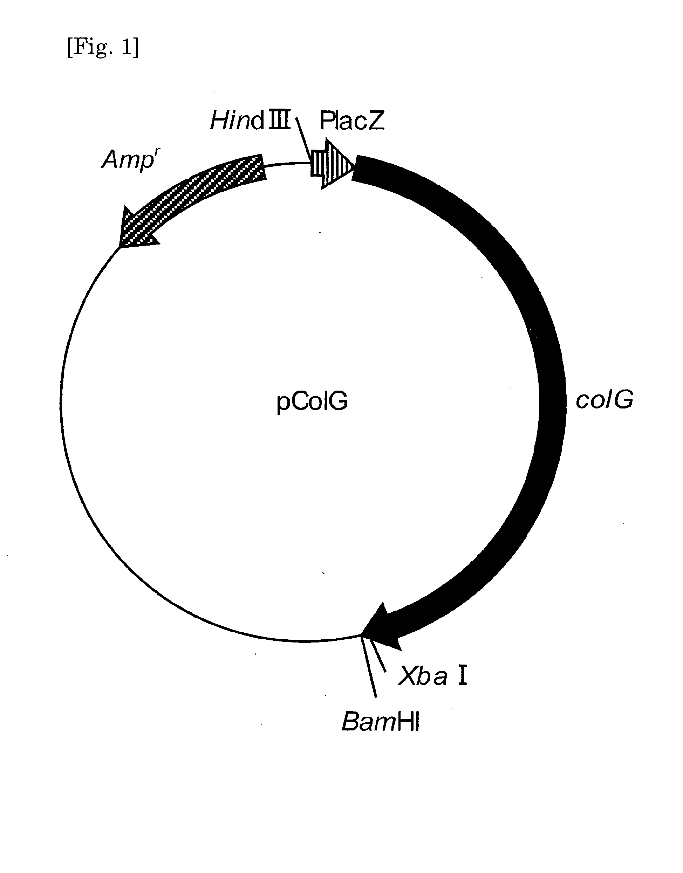

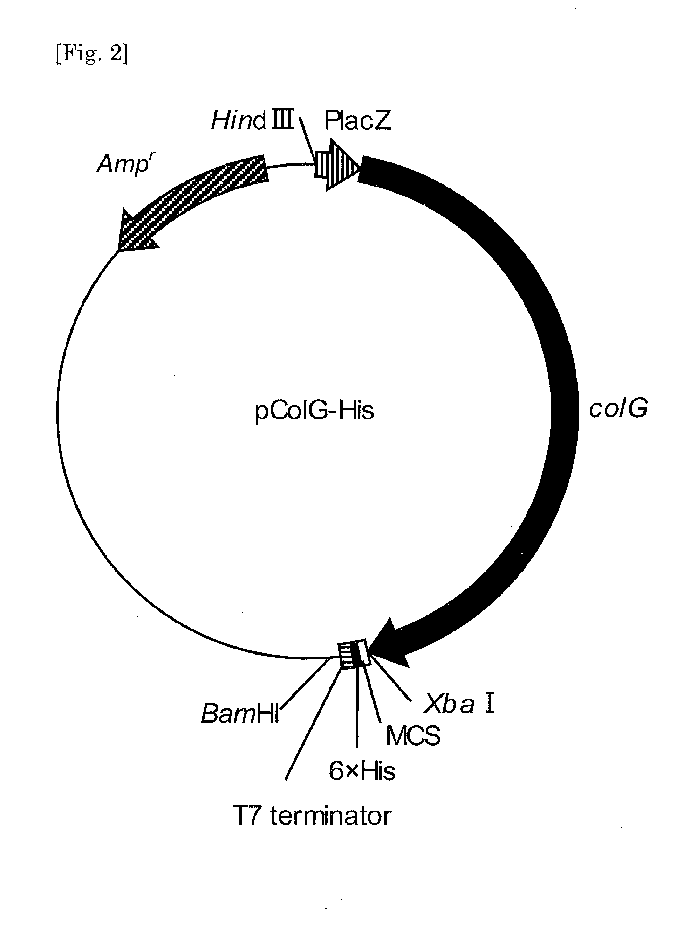

Three kinds of DNA fragments prepared by the method described above were inserted into pBR322, a commercially available vector, so as to link them in order of the lacZ promoter, the collagenase G gene, and the DNA fragment including the histidine tag from the 5' end. First, the lacZ promoter and the collagenase G gene were inserted into pBR322. Specifically, the lacZ promoter region and the collagenase G gene fragment prepared as described above were phosphorylated, and then inserted into pBR322 that had been double-digested with HindIII and BamHI to construct pColG (Fig. 1). It is to be noted that the lacZ gene promoter region (PlacZ) and the colG gene were linked to each other at their blunt ends. Subsequently, the DNA encoding the histidine tag prepared by the method described above was inserted into pColG that had been double-digested with XbaI and BamHI to construct pColG-His (Fig. 2). The DNA that was finally inserted into pBR322 was a DNA having the base sequence from positions 1 to 3396 of Sequence Listing: 4.

### (1-5) Preparation of E. coli expressing fusion collagenase G

pColG-His was transformed into E. coli, Escherichia coli strain χ1776, according to the conventional method, and cultured at 37°C for a full day on LB agar to which 20 µg/ml of diaminopimelic acid, 100 µg/ml of thymidine, and 50 µg/ml of ampicillin had been added. Thus, E. coli expressing the fusion collagenase G was prepared.

### [Example 2] Culturing of E. coli expressing fusion collagenase G, and selective collection of collagenase G having CBD

### (2-1) Culturing of E. coli expressing fusion collagenase G

The E. coli expressing the fusion collagenase G obtained in Example 1 was inoculated in a 250-ml Erlenmeyer flask with 100 ml of a medium being added, and cultured with stirring at 200 rpm at 28°C for 16 hours. The medium used in this culturing was a TB medium (1.2% triptone, 2.4% yeast extract, 0.94% dipotassium hydrogenphosphate, 0.22% potassium dihydrogenphosphate, 0.8% glycerol) to which 100 µg/ml of diaminopimelic acid, 20 µg/ml of thymidine, 50 µg/ml of ampicillin, and 0.1 mM of IPTG was added.

### (2-2) Preparation of extract of E. coli expressing fusion collagenase G

The resulting culture solution obtained in (2-1) was subjected to centrifugation to collect cells, followed by lysis of the collected cells in 10 ml of POP culture Regent (manufactured by Merck & Co., Inc.) to extract a protein in the cells. The supernatant obtained by centrifugation of the lysate was filtrated with a 0.2-µm membrane to remove a gene recombinant, thereby providing an extract of the E. coli expressing the fusion collagenase G.

### (2-3) Selective collection of fusion collagenase G having CBD by affinity chromatography

To remove a degraded collagenase from the extract of the E. coli expressing the fusion collagenase G obtained in (2-2), the extract was fractionated by a nickel chelate column which was affinity chromatography for the histidine tag. To 10 ml of the extract of the E. coli expressing the fusion collagenase G prepared by the method described above, 60 ml of a buffer (20 mM sodium phosphate buffer (pH 7.5) to which 0.5 M NaCl and 20 mM imidazole were added) for nickel chelate column binding was added. The mixture was passed through a 100-ml nickel chelate column equilibrated with a buffer for nickel chelate column binding. Then, the column was washed with a suitable amount of a buffer for nickel chelate column binding to remove a degraded collagenase that were not able to be adsorbed to the nickel chelate column. Thereafter, 100 ml of a buffer for nickel chelate column binding to which 500 mM imidazole had been added was passed through the column. Thus, the fusion collagenase G having the CBD was collected.

### (2-4) Confirmation of removal of degraded collagenase

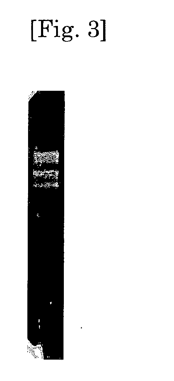

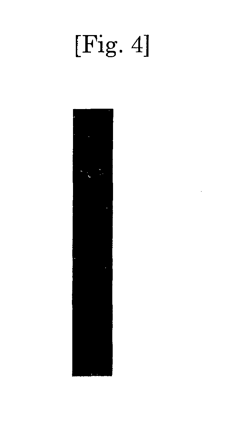

To confirm that the degraded collagenase was removed from the extract of the fusion collagenase G, the activity staining was performed on the extract of the E. coli expressing the fusion collagenase G and the solution of the fusion collagenase G subjected to the affinity chromatography. The activity staining was performed on 0.25 µl of the extract of the E. coli expressing the fusion collagenase G obtained in (2-2) and 2.5 µl of the solution of the fusion collagenase G subjected to the affinity chromatography obtained in (2-3) with Zymogram-PAGE mini (manufactured by TEFCO). As a result, five bands indicating protease activity were observed for the extract of the E. coli expressing the fusion collagenase G (Fig. 3). On the other hand, for the solution of the fusion collagenase G subjected to the affinity chromatography, one band indicating the maximum molecular weight was observed as a main band among the above five bands (Fig. 4). Based on the comparison and the analysis of these bands, it was found that part or all of the CBD was degraded when the fusion collagenase G was expressed by E. coli. The foregoing results showed that the fusion collagenase having the CBD was successfully selectively collected by purifying the extract of the E. coli expressing the fusion collagenase G by the affinity chromatography and thereby removing the collagenase in which part or all of the CBD was degraded.

### [Example 3] Expression of fusion collagenase in which histidine tag is linked to carboxyl terminal of collagenase H derived from Clostridium histolyticum

### (3-1) Preparation of collagenase H gene fragment

A collagenase H gene derived from Clostridium histolyticum was amplified from the 5' end to the 3' end thereof by PCR with a template of a genomic DNA derived from Clostridium histolyticum. In this case, the primers were designed so that the 3' end of the amplified collagenase H gene served as an XbaI-recognition sequence and further that a BamHI-recognition sequence was added subsequent to the stop codon of the gene. As a result, a mutation was introduced into the amino acid sequence at one position (an amino acid at position 980 of SEQ ID NO: 5) on the carboxyl terminal side of the amino acid sequence of natural collagenase H described in Non-Patent Literature 2. Thus, the amino acid sequence was modified to the amino acid sequence of SEQ ID NO: 6.

The primers used in this PCR were as follows.
colH-F:
   ATGAAAAGGAAATGTTTATC (SEQ ID NO: 15)
colH-R:
   CCGGATCCTATCTAGATACTGAACCTT (SEQ ID NO: 16)

The amplified collagenase H gene fragment was digested with BamHI.

### (3-2) Preparation of DNA fragment including region encoding histidine tag

A DNA fragment including the region encoding the histidine tag was prepared by the same method as in Example 1.

### (3-3) Preparation of lacZ promoter fragment

A lacZ promoter fragment was prepared by the same method as in Example 1.

### (3-4) Construction of expression vector for fusion collagenase H to which histidine tag is linked

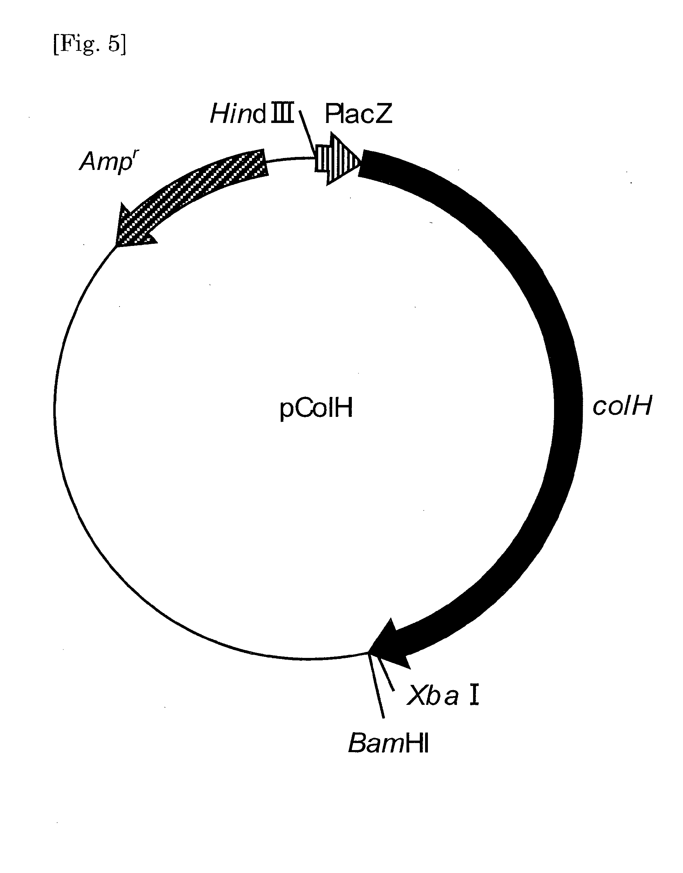

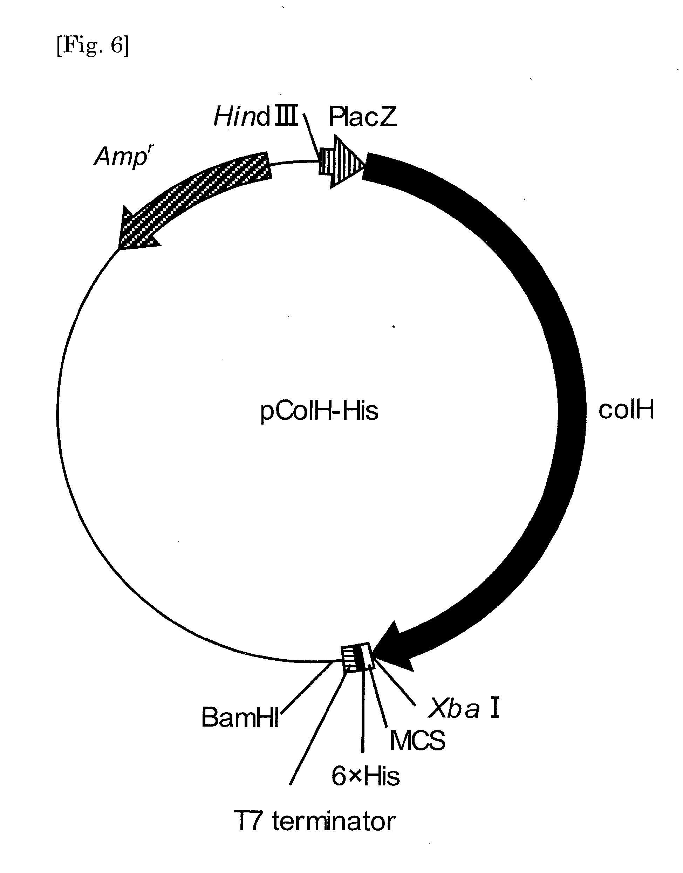

Three kinds of DNA fragments prepared by the method described above were inserted into pBR322, a commercially available vector, so as to link them in order of the lacZ promoter, the collagenase H gene, and the DNA fragment including the histidine tag from the 5' end. First, to insert the lacZ promoter and the collagenase H gene into pBR322, the lacZ promoter region and the collagenase H gene fragment prepared as described above were phosphorylated, and then inserted into pBR322 that had been double-digested with HindIII and BamHI to construct pColH (Fig. 5). It is to be noted that the lacZ gene promoter region and the collagenase H gene were linked to each other at their blunt ends. Subsequently, the DNA fragment including the DNA encoding the histidine tag prepared by the method described above was inserted into pColH that had been double-digested with XbaI and BamHI to construct pColH-His (Fig. 6). The DNA that was finally inserted into pBR322 was a DNA having the base sequence from positions 1 to 3105 of Sequence Listing: 8.

### (3-5) Preparation of E. coli expressing fusion collagenase H

E. coli expressing the fusion collagenase H was created by the same method as in Example 1.

### [Example 4] Culturing of E. coli expressing fusion collagenase H, and selective collection of collagenase H having CBD

### (4-1) Culturing of E. coli expressing fusion collagenase H

The E. coli expressing the fusion collagenase H obtained in Example 3 was cultured by the method described in Example 2 to obtain a culture solution.

### (4-2) Preparation of extract of E. coli expressing fusion collagenase H

An extract of the E. coli expressing the fusion collagenase H was obtained from the resulting culture solution obtained in (4-1) by the method described in Example 2.

### (4-3) Selective collection of fusion collagenase H having CBD by affinity chromatography

The extract of the E. coli expressing the fusion collagenase H obtained in (4-2) was subjected to affinity chromatography by the method described in Example 2 to thus collect the fusion collagenase H having the CBD.

### (4-4) Confirmation of removal of degraded collagenase

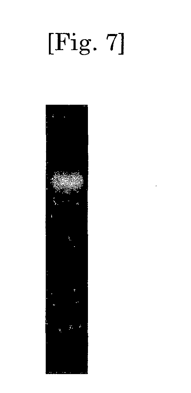

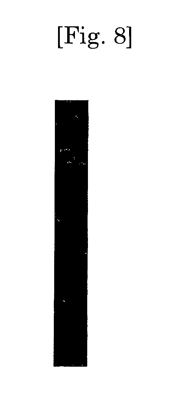

To confirm how much the degraded collagenase was removed from the fusion collagenase H, the activity staining was performed after electrophoresis by the method described in Example 2. As a result, four bands indicating protease activity were observed for the extract of the E. coli expressing the fusion collagenase H (Fig. 7). On the other hand, for the solution of the fusion collagenase H subjected to the affinity chromatography, one band indicating the maximum molecular weight was observed as a main band among the above four bands (Fig. 8). Based on the comparison and the analysis of these bands, it was found that part or all of the CBD in the fusion collagenase H expressed by E. coli was degraded. The foregoing results showed that the fusion collagenase H having the CBD was successfully selectively collected by purifying the extract of the E. coli expressing the fusion collagenase H by the affinity chromatography and thereby removing the collagenase in which part or all of the CBD was degraded.

### [Industrial Applicability]

According to the present invention, a collagenase as a recombinant protein can be produced with high purity and without including a degraded product caused by an action of a host. The use of a collagenase produced by the method of the present invention allows, for example, pancreatic islets to be separated from a pancreas without decreasing the quality of the pancreatic islets, thereby greatly contributing to pancreatic islet transplantation to diabetic patients.
## Claims

1. A method for producing a fusion collagenase,
wherein:
a culture obtained by culturing E. coli transformed by any one of a DNA encoding the fusion collagenase and an expression vector comprising the DNA is purified by affinity chromatography corresponding to an affinity tag to thereby selectively collect the fusion collagenase having a collagen-binding domain, wherein the fusion collagenase has the following features (i) to (ii):
(i)the affinity tag is directly or indirectly linked to a carboxyl terminal of the collagenase;
(ii) the collagenase is selected from the following (a) to (h) :
(a) a collagenase comprising an amino acid sequence from positions -110 to 1008 of any one of SEQ ID NOS: 1 and 2;
(b) a collagenase comprising an amino acid sequence from positions -110 to 1008 of any one of SEQ ID NOS: 1 and 2 in which 1 to 30 amino acids are deleted, substituted, inserted, or added;
(c) a collagenase comprising an amino acid sequence having a homology of 80% or more with the amino acid sequence from positions -110 to 1008 of any one of SEQ ID NOS: 1 and 2;
(d) a collagenase in which an amino acid sequence from positions -110 to -1 of any one of SEQ ID NOS: 1 and 2 is removed from any one of the collagenases described in (a) to (c);
(e) a collagenase comprising an amino acid sequence from positions -40 to 981 of any one of SEQ ID NOS: 5 and 6;
(f) a collagenase comprising an amino acid sequence from positions -40 to 981 of any one of SEQ ID NOS: 5 and 6 in which 1 to 30 amino acids are deleted, substituted, inserted, or added;
(g) a collagenase comprising an amino acid sequence having a homology of 80% or more with the amino acid sequence from positions -40 to 981 of any one of SEQ ID NOS: 5 and 6;
(h) a collagenase in which an amino acid sequence from positions -40 to -1 of any one of SEQ ID NOS: 5 and 6 is removed from any one of the collagenases described in (e) to (g).

2. The method for producing according to claim 1, wherein the fusion collagenase is a fusion collagenase in which the affinity tag is two or more consecutive histidine residues.

3. The method for producing according to claim 1, wherein the fusion collagenase comprises an amino acid sequence from positions -110 to 1021 of SEQ ID NO: 3.

4. The method for producing according to claim 3, wherein the fusion collagenase is a fusion collagenase from which an amino acid sequence from positions -110 to -1 of SEQ ID NO: 3 is removed.

5. The method for producing according to claim 1, wherein the fusion collagenase comprises an amino acid sequence from positions -40 to 994 of SEQ ID NO: 7.

6. The method for producing according to claim 5, wherein the fusion collagenase is a fusion collagenase from which an amino acid sequence from positions -4 0 to -1 of SEQ ID NO: 7 is removed.

7. The method for producing according to claim 1, wherein the DNA encoding the fusion collagenase is a DNA comprising a base sequence from positions 1 to 3396 of SEQ ID NO: 4.

8. The method for producing according to claim 1, wherein the DNA encoding the fusion collagenase is a DNA comprising a base sequence from positions 1 to 3105 of SEQ ID NO: 8.

## Patentansprüche

1. Verfahren zur Herstellung einer Fusions-Collagenase, worin eine durch Kultivierung von E. coli erhaltene Kultur, die von einer die Fusions-Collagenase kodierenden DNA und einem die DNA umfassenden Expressionsvektor transformiert wurde, durch eine Affinitätschromatographie mit einem korrespondierenden Affinitäts-Tag gereinigt wird, um dadurch selektiv die Fusions-Collagenase mit einer Collagen bindenden Domäne zu gewinnen, worin die Fusions-Collagenase folgende Merkmale (i) bis (ii) aufweist:
(i) das Affinitäts-Tag ist direkt oder indirekt an einen Carboxylterminus der Collagenase gebunden;
(ii) die Collagenase ist ausgewählt aus den folgenden (a) bis (h):
(a) eine Collagenase, umfassend eine Aminosäuresequenz von Position -110 bis 1008 einer der SEQ ID NOS: 1 und 2;
(b) eine Collagenase, umfassend eine Aminosäuresequenz von Position -110 bis 1008 einer der SEQ ID NOS: 1 und 2, in denen 1 bis 30 Aminosäuren entfernt, substituiert, eingefügt oder hinzugefügt sind;
(c) eine Collagenase, umfassend eine Aminosäuresequenz mit eine Homologie von 80 % oder mehr mit der Aminosäuresequenz von Position -110 bis 1008 einer der SEQ ID NOS: 1 und 2;
(d) eine Collagenase, in der die Aminosäuresequenz von Position -110 bis -1 einer der SEQ ID NOS: 1 und 2 von einer der in (a) bis (c) beschriebenen Collagenase entfernt wurde;
(e) eine Collagenase, umfassend eine Aminosäuresequenz von Position -40 bis 981 einer der SEQ ID NOS: 5 und 6;
(f) eine Collagenase, umfassend eine Aminosäuresequenz von Position -40 bis 981 einer der SEQ ID NOS: 5 und 6, in der die Aminosäuren 1 bis 30 entfernt, substituiert, eingefügt oder hinzugefügt wurden;
(g) eine Collagenase, umfassend eine Aminosäuresequenz mit einer Homologie von 80 % oder mehr mit der Aminosäuresequenz von Position -40 bis 981 einer der SEQ ID NOS: 5 und 6;
(h) eine Collagenase, in der eine Aminosäuresequenz von Position -40 bis -1 einer der SEQ ID NOS: 5 und 6 von einer der in (e) bis (g) beschriebenen Collagenasen entfernt wurde.

2. Das Verfahren nach Anspruch 1, worin die Fusions-Collagenase eine Fusions-Collagenase ist, in der das Affinitäts-Tag aus zwei oder mehr konsekutiven Histidin-Einheiten besteht.

3. Das Verfahren nach Anspruch 1, worin die Fusions-Collagenase eine Aminosäuresequenz von Position -110 bis 1021 der SEQ ID NO: 3 umfasst.

4. Das Verfahren nach Anspruch 3, worin die Fusions-Collagenase eine Fusions-Collagenase ist, von der eine Aminosäuresequenz der Position -110 bis -1 der SEQ ID NO: 3 entfernt ist.

5. Das Verfahren nach Anspruch 1, worin die Fusions-Collagenase eine Aminosäuresequenz von Position -40 bis 994 der SEQ ID NO: 7 umfasst.

6. Das Verfahren nach Anspruch 5, worin die Fusions-Collagenase eine Fusions-Collagenase ist, von der eine Aminosäuresequenz von Position -40 bis -1 der SEQ ID NO: 7 entfernt ist.

7. Das Verfahren nach Anspruch 1, worin die die Fusions-Collagenase kodierende DNA, eine DNA ist, die eine Basensequenz von Position 1 bis 3396 der SEQ ID NO: 4 umfasst.

8. Das Verfahren nach Anspruch 1, worin die die Fusions-Collagenase kodierende DANN, eine DNA ist, die eine Basensequenz von Position 1 bis 3105 der SEQ ID NO: 8 umfasst.

## Revendications

1. Procédé de production d'une collagénase de fusion, dans lequel :
une culture obtenue par culture de *E. coli* transformé par un ADN codant la collagénase de fusion ou un vecteur d'expression comprenant l'ADN, est purifiée par chromatographie d'affinité à l'aide d'une étiquette d'affinité pour ainsi récolter sélectivement la collagénase de fusion ayant un domaine liant la collagénse, où la collagénase de fusion présente les caractéristiques (i) à (ii) suivantes :
(i) l'étiquette d'affinité est liée directement ou indirectement l'extrémité carboxyle de la collagénase ;
(ii) la collagénase est choisie parmi les (a) à (h) suivantes :
(a) une collagénase comprenant la séquence des acides aminés allant des positions -110 à 1008 de l'une quelconque des SEQ ID N°1 et 2 ;
(b) une collagénase comprenant la séquence des acides aminés allant des positions -110 à 1008 de l'une quelconque des SEQ ID N°1 et 2, dans laquelle 1 à 30 acides aminés sont délétés, substitués, insérés ou ajoutés ;
(c) une collagénase comprenant la séquence des acides aminés ayant 80% d'homologie ou plus avec la séquence des acides aminés allant des positions -110 à 1008 de l'une quelconque des SEQ ID N°1 et 2 ;
(d) une collagénase dans laquelle la séquence des acides aminés allant des positions -110 à -1 de l'une quelconque des SEQ ID N°1 et 2 est éliminée de l'une quelconque des collagénase décrites en (a) à (c) ;
(e) une collagénase comprenant la séquence des acides aminés allant des positions -40 à 98 de l'une quelconque des SEQ ID N°5 et 6 ;
(f) une collagénase comprenant la séquence des acides aminés allant des positions -40 à 981 de l'une quelconque des SEQ ID N°5 et 6, dans laquelle 1 à 30 acides aminés sont délétés, substitués, insérés ou ajoutés ;
(g) une collagénase comprenant la séquence des acides aminés ayant 80% d'homologie ou plus avec la séquence des acides aminés allant des positions -40 à 981 de l'une quelconque des SEQ ID N°5 et ;
(h) une collagénase dans laquelle la séquence des acides aminés allant des positions -40 à -1 de l'une quelconque des SEQ ID N°5 et 6 est éliminée de l'une quelconque des collagénase décrites en (e) à (g).

2. Procédé de production selon la revendication 1, où la collagénase de fusion est une collagénase de fusion dans laquelle l'étiquette d'affinité consiste en deux résidus histidine consécutifs ou plus.

3. Procédé de production selon la revendication 1, où la collagénase de fusion comprend une séquence des acides aminés des positions -110 à 1021 de SEQ ID N°3.

4. Procédé de production selon la revendication 3, où la collagénase de fusion est une collagénase de fusion de laquelle la séquence des acides aminés allant des positions -110 à -1 de SEQ ID N°3 est éliminée.

5. Procédé de production selon la revendication 1, où la collagénase de fusion comprend une séquence des acides aminés des positions -40 à 994 de SEQ ID N°7.

6. Procédé de production selon la revendication 5, où la collagénase de fusion est une collagénase de fusion de laquelle la séquence des acides aminés allant des positions -40 à -1 de SEQ ID N°7 est éliminée.

7. Procédé de production selon la revendication 1, où l'ADN codant la collagénase de fusion est un ADN comprenant la séquence des bases des positions 1 à 3396 de SEQ ID N°4.

8. Procédé de production selon la revendication 1, où l'ADN codant la collagénase de fusion est un ADN comprenant la séquence des bases des positions 1 à 3105 de SEQ ID N°8.
